# EUROPEAN PATENT APPLICATION

(11) **EP 2 025 313 A1**
(43) Date of publication of application: **18.02.2009**
(21) Application number: 07744591.4
(22) Date of filing: 01.06.2007
(51) Int. Cl.: A61F 13/56, A61F 13/15, A61F 13/514

(54) **ABSORBENT ARTICLE**

(30) Priority: 02.06.2006 JP 2006155114
(71) Applicant: Uni-Charm Corporation, Ehime 799-0111 (JP)
(72) Inventor: NODA, Yuki, Kanonji-shi, Kagawa 769-1602 (JP); KURODA, Kenichiro, Kanonji-shi, Kagawa 769-1602 (JP)
(74) Representative: Fitchett, Stuart Paul
(86) International application number: PCT/JP2007/061204
(87) International publication number: WO 2007/142145

(57) **Abstract**

An absorbent article that by sticking to the excretory part of the wearer without slippage of sanitary napkin, prevents any leakage of excrements, such as body fluid. The absorbent article is one comprising an absorbent article main body including at least surface sheet part (2) whose at least one area is permeable for liquid, disposed on one side in the direction of the thickness of the absorbent article and liquid-retaining absorbent part (4) disposed opposite to the surface sheet part (2) on the other side in the direction of the thickness of the absorbent article, and comprising, disposed on the other side of the absorbent article main body, belt member (10) arranged so as to extend along the longitudinal direction (LD,LD) of the absorbent article main body, the absorbent article shaped substantially longitudinally long. The belt member (10) on its side of at least one end of both ends thereof in the longitudinal direction (LD,LD) is provided with locking part (9f,9r).

## Description

### BACKGROUND ART

Sanitary napkins, panty liners, urine-absorbing pads, and the like have conventionally been used as an absorbent article for absorbing excrement such as menstrual blood. These absorbent articles have an absorbent core for absorbing and holding menstrual blood or the like, a liquid permeable top sheet that covers the surface on a skin contacting surface of the absorbent core, and a liquid impermeable back sheet that covers the back positioned on the clothing side of the absorbent core. For example, these absorbent articles can be adhered to the internal surface of a crotch piece of underwear.

Incidentally, in order to trap the excrement such as menstrual blood, it is desired that the abovementioned absorbent articles be used in a state where the absorption layer having the absorbent body is in contact with the excretory part of a wearer. However, since the absorbent articles are by nature adapted for use in a state where these are attached to underwear or the like, both are susceptible to relative dislocation between the underwear and the excretory part. Moreover, if there is a space between the excretory part and the absorbent article, the excrement dropped on the top sheet may effuse along the top sheet toward the sides and the buttocks, resulting in soiling of the underwear and clothing.

On the other hand, for example, Japanese Utility Model Application Publication No. Hei 03-101933 (hereinafter referred to as Patent Document 1) discloses an absorbent article having improvements in adhesion (adhesive properties) of the absorbent article to the wearer's body while wearing underwear. More specifically, flexible flaps are formed on both ends in the longitudinal direction of the absorbent article, and retainers provided at the flaps can be adhered to the underwear.

In addition, for example, Japanese Patent Application Publication No. Hei 11-99179 (hereinafter referred to as Patent Document 2) discloses an absorbent article having improvements in adhesion of the absorbent article to the wearer's body when underwear is put on; more specifically, a flexible elastic member can be extended from the edge portion of a sanitary napkin, and an adhesive region is formed on a contact surface with the underwear located at the portion so extended, and then adhered to the underwear or the like.

Each of the absorbent articles as disclosed in Patent Documents 1 and 2 is provided with the elastic portions on both ends of the absorbent article in the longitudinal direction, and adapted to improve adhesion by pulling the absorbent article toward the excretory part of the wearer by the elastic force generated in the elastic members.

Furthermore, for example, Japanese Patent Application Publication No. 2005-95510 (hereinafter referred to as Patent Document 3) discloses an absorbent article having a hole or a guide portion through which a belt passes, where the belt has a fixing portion in each end, for fixing the belt to a top sheet and the like of the absorbent article.

The absorbent articles disclosed in Patent Documents 1, 2 and 3 are intended to prevent absorbent article dislocation and the like by providing a belt passing through a hole or a guide portion provided on a back surface sheet of the absorbent article.

### DISCLOSURE OF THE INVENTION

### Problems to be Solved by the Invention

However, since the absorbent articles disclosed in Patent Documents 1 and 2 have a configuration pulling the absorbent article back and forth by the elastic force of the elastic members provided on both ends of the absorbent article, sufficient stress might not be obtained to contact the absorbent article with the excretory part. For example, in a case where the absorbent article is deformed in a concaved shape with respect to the wearer's excretory part due to a lateral force applied from the wearer's thigh, a gap is generated between the wearer's body and the absorbent article due to low adhesion of the absorbent article to the wearer's excretory part.

In addition, the belt of the absorbent article disclosed in Patent Document 3 merely passes through the guide portion and is not fixed to the absorbent article. The belt can thus be dislocated from the absorbent article while wearing. In addition, in a case where the belt is fixed to the absorbent article in positions spaced apart from each other, even a stretchable belt cannot achieve stretchability and the absorbent article does not follow the belt in a portion between the positions.

The present invention has been made in view of the foregoing problems and aims at providing an absorbent article that prevents leakage of excrement, such as bodily fluid, by improving the adhesion between the excretory part or the like of the wearer and the absorbent article.

### Means for Solving the Problems

To this end, the present inventors have achieved the present invention based on the discovery that stress toward a skin-contacting side, generated on a belt-shaped member, can be transferred to the absorbent article main body, by arranging the belt-shaped member along a longitudinal direction of the absorbent article, on a skin-noncontacting side thereof and by engaging both ends of the belt-shaped member with a target object. More specifically, the present invention provides the following absorbent article.

In a first aspect of the present invention, an absorbent article, having a substantially elongated shape, includes: an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and a belt-shaped member disposed on the second side of the absorbent article main body, along a longitudinal direction of the absorbent article main body, in which an engaging portion is provided in at least one end side of both ends in the longitudinal direction of the belt-shaped member.

According to the first aspect of the present invention, the absorbent article includes an absorbent article main body including a top sheet portion that is at least partially liquid permeable and disposed on a first side in a thickness direction of the absorbent article and a liquid retentive absorbent core portion disposed on a second side in the thickness direction of the absorbent article, in other words on an opposite side of the top sheet portion and a belt-shaped member disposed on the second side of the absorbent article main body, along a longitudinal direction of the absorbent article main body, and has a substantially elongated shape. An engaging portion is provided in at least one end side of both ends in the longitudinal direction of the belt-shaped member.

Therefore, in the absorbent article, the belt-shaped member is arranged along the longitudinal direction of the absorbent article main body, and both end portions of the belt-shaped member are respectively fixed or engaged to a predetermined position of a target object such as underwear. Thus, the entire absorbent article main body can be lifted toward the wearer's body. This improves the adhesion between the wearer's excretory part or the like and the absorbent article, enabling prevention of leakage of excrement such as menstrual blood.

According to a second aspect of the present invention, in the absorbent article of the first aspect, the belt-shaped member is formed to be at least partially extensible.

According to the second aspect of the present invention, the belt-shaped member is formed to be at least partially extensible. The extensibility of the belt-shaped member can ensure, for example, a predetermined length of the belt-shaped member necessary for the attachment to underwear as a target object. In addition, since the belt-shaped member is not extended when it is not attached thereto, it is possible to reduce the size of the belt-shaped member in the absorbent article.

The term "formed to be extensible" indicates, for example, applying a corrugated embossing finish or forming predetermined folds on the belt-shaped member. Alternatively, an extensible base material sheet may be used.

According to a third aspect of the present invention, in the absorbent article of the first or the second aspect, the belt-shaped member is formed to be at least partially stretchable.

According to the third aspect of the present invention, the belt-shaped member is formed to be at least partially stretchable. The stretchability of the belt-shaped member can maintain adherence of the absorbent article main body to the wearer's body. This is because, for example, even if a relative dislocation occurs between underwear and the wearer's body due to loosening of the underwear or deformation thereof following movement of the wearer's body, the belt-shaped member can expand and contract following the dislocation. This improves the adhesion between the wearer's excretory part and the like and the absorbent article, thereby preventing leakage of excrement such as menstrual blood.

According to a fourth aspect of the present invention, in the absorbent article of the second or the third aspect, a length of the belt-shaped member in an extended state thereof is greater than a length in the longitudinal direction of the absorbent article main body.

According to the fourth aspect of the present invention, a length of the belt-shaped member in an extended state thereof is greater than a length in the longitudinal direction of the absorbent article main body. The belt-shaped member can thus have a predetermined length for lifting the absorbent article main body.

According to a fifth aspect of the present invention, in the absorbent article of the second or the third aspect, a length of the belt-shaped member in a non-extended state thereof is greater than a length in the longitudinal direction of the absorbent article main body.

According to the fifth aspect of the present invention, the belt-shaped member, in a non-extended state, is formed so as to be longer than a length in the longitudinal direction of the absorbent article main body. With such a configuration, both ends of the belt-shaped member can be kept extended from the absorbent article main body, thus allowing the wearer to easily grip the both ends of the belt-shaped member. This can improve operability of the absorbent article.

According to a sixth aspect of the present invention, in the absorbent article of any one of the first to the fifth aspects, a grip portion, of which at least a part extends from an outer peripheral portion in both end sides of the absorbent article main body in the longitudinal direction, is formed in each of the both end sides of the belt-shaped member in the longitudinal direction.

According to the sixth aspect of the present invention, a grip portion, which extends from an outer peripheral portion in both ends of the absorbent article main body in the longitudinal direction, is formed in both ends, respectively, of the belt-shaped member in the longitudinal direction. This allows the wearer to wear the absorbent article using the grip portion, thus improving the comfort thereof.

According to a seventh aspect of the present invention, in the absorbent article as described in any one of the first to the sixth aspects, a cover member is further provided on the second side of the absorbent article main body, which encapsulates at least a part in the longitudinal direction of the belt-shaped member and an entirety in the width direction of the belt-shaped member.

According to the seventh aspect of the present invention, the absorbent article includes, on the second side in the thickness direction, a cover member for wrapping the belt-shaped member. The cover member wraps at least a part in the longitudinal direction of the belt-shaped member and an entirety in the width direction of the belt-shaped member. That is, the cover member is provided so as to wrap the absorbent article in the width direction on the second side thereof, and the belt-shaped member is disposed between the absorbent article and the cover member. At this time, the belt-shaped member is arranged so as to be slidable between the absorbent article main body and the cover member, and can be extended from an opening formed between the absorbent article main body and the cover member on both ends in the longitudinal direction. As described above, by providing the cover member wrapping the belt-shaped member, the belt-shaped member can be kept clean. The cover member is also capable of indicating the sliding direction of the belt-shaped member. Furthermore, it should be noted that the cover member may wrap a part or an entirety of the belt-shaped member in the longitudinal direction. Alternatively, a plurality of the cover members may be provided in the longitudinal direction.

According to an eighth aspect of the present invention, in the absorbent article of any one of the first to the seventh aspects,the belt-shaped member joins the absorbent article main body in a predetermined position on the second side of the absorbent article.

According to the eighth aspect of the present invention, the belt-shaped member joins the absorbent article in a predetermined position on the second side thereof. This prevents dislocation of the belt-shaped member from the absorbent article. In addition, in a case where the belt-shaped member is fixed in a plurality of positions, by applying stretchability to the absorbent article main body between the positions, the absorbent article can still follow the wearer's body.

According to a ninth aspect of the present invention, in the absorbent article of any one of the first to the eighth aspect, the grip portion has a guiding element for implying an extension direction of the belt-shaped member.

According to the ninth aspect of the present invention, the grip portion has a guiding element for implying an extension direction of the belt-shaped member. For example, the guiding element can direct the belt-shaped member to a predetermined direction when wearing. This enables the wearer to confirm an appropriate manner of wearing by referring to the guiding element. In addition, even in a case where the guiding element cannot be visually confirmed, the guiding element can be formed so as to be confirmed by touch, thereby preventing the wearer from wearing the absorbent article in the wrong orientation.

According to a tenth aspect of the present invention, in the absorbent article described in any one of the first to the ninth aspects, a predetermined engaging portion is disposed in each of the both ends of the belt-shaped member in the longitudinal direction.

According to the tenth aspect of the present invention, a predetermined engaging portion is disposed in both ends, respectively, of the belt-shaped member in the longitudinal direction. This enables each of the engaging portions to be engaged to a predetermined position of a target object such as underwear. Therefore, adhesion between the wearer's excretory part and the like and the absorbent article is improved, thereby enabling prevention of leakage of excrement such as menstrual blood.

According to an eleventh aspect of the present invention, in the absorbent article of any one of the first to the tenth aspects, the belt-shaped member includes at least one portion that is a liquid impermeable material.

According to the eleventh aspect of the present invention, the belt-shaped member includes at least one portion that is a liquid impermeable material. Thus, even without a liquid impermeable back surface sheet portion used in the absorbent article main body, for example, it is possible to prevent leakage to another side of the absorbent article by disposing the belt-shaped member on the other side of the absorbent core portion.

According to a twelfth aspect of the present invention, in the absorbent article of any one of the first to the eleventh aspects, a length of the belt-shaped member in a width direction is at least 30% of a length of the absorbent article main body in a width direction.

According to the twelfth aspect of the present invention, a length of the belt-shaped member in a width direction is at least 30% of a length of the absorbent article main body in a width direction. Thus, the belt-shaped member can be brought into contact with a predetermined range of the absorbent article main body in the width direction and, for example, the entirety of the absorbent article main body can be lifted while maintaining an appropriate shape. This enables the absorbent article main body to be adhered to a predetermined portion of the wearer's body, thereby enabling the prevention of the leakage of excrement such as menstrual blood.

According to a thirteenth aspect of the present invention, in the absorbent article described in any one of the first to the twelfth aspects, the belt-shaped member includes a belt-shaped base material and an elastic member.

According to the thirteenth aspect of the present invention, the belt-shaped member includes a belt-shaped base material and an elastic member. This enables the prevention of so-called neck in, for example, which could be caused when a belt-shaped elastic member is used, namely a state in which the substantially central portion thereof becomes narrow.

In a fourteenth aspect of the present invention, an absorbent article having a substantially elongated shape, which is used in contact with a wearer's body by being placed on an inner surface of a predetermined clothing, includes: an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and a belt-shaped member disposed on the second side of the absorbent article main body, along a longitudinal direction of the absorbent article main body; and an engaging portion provided in at least one end side of both ends in the longitudinal direction of the belt-shaped member, in which the belt-shaped member pushes up the absorbent article main body to the first side during wearing, by being extended and engaging the engaging portion to a target object.

According to the fourteenth aspect of the present invention, the absorbent article includes: an absorbent article main body including a top sheet portion that is at least partially liquid permeable and disposed on a first side in a thickness direction and a liquid retentive absorbent core portion disposed on a second side in the thickness direction of the absorbent article, i.e. on an opposite side of the top sheet portion; a belt-shaped member disposed on the second side of the absorbent article main body, along a longitudinal direction of the absorbent article main body; and an engaging portion provided in at least one end side of both ends in the longitudinal direction of the belt-shaped member. The belt-shaped member is used in the state where it is extended and engaged with, for example, the internal surface of predetermined clothing such as underwear.

This enables the belt-shaped member to press the absorbent article main body upwards along a predetermined portion of the wearer. For example, it is possible to attain the state of lifting along the curvature of the wearer's body. Furthermore, the stress generated in the belt-shaped member can be directed from the entirety of the absorbent article main body to a predetermined body part of the wearer, enabling the entire absorbent article main body to be in contact with the predetermined body part of the wearer. The predetermined body part is, for example, an excretory part of the wearer.

In a fifteenth aspect of the present invention, an absorbent article having a first mode of usage and a second mode of usage is provided, includes: an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; a belt-shaped member disposed on the second side of the absorbent article main body, along a longitudinal direction of the absorbent article main body; and an engaging portion provided in at least one end side of both ends in the longitudinal direction of the belt-shaped member, in which in the first mode of usage, the absorbent article is used with the belt-shaped member being engaged to a target object; and in the second mode of usage, the absorbent article is used with the belt-shaped member not being engaged to the target object.

According to the fifteenth aspect of the present invention, the absorbent article includes: an absorbent article main body including a top sheet portion that is at least partially liquid permeable and disposed on a first side in a thickness direction and a liquid retentive absorbent core portion disposed on a second side in the thickness direction of the absorbent article, i.e. on an opposite side of the top sheet portion; a belt-shaped member that is liquid permeable disposed between the top sheet portion and the absorbent core portion in a longitudinal direction of the absorbent article main body; and an engaging portion provided in at least one end side of both ends in the longitudinal direction of the belt-shaped member. The absorbent article has a first mode of usage in which the belt-shaped member is engaged with a target object such as underwear or the wearer's buttocks, and a second mode of usage in which the belt-shaped member is not engaged with the target object.

In this way, the absorbent article may be used by either engaging or not engaging the belt-shaped member with predetermined underwear or the like. Therefore, depending on the situation of the wearer, this allows a mode of usage to be selected.

According to a sixteenth aspect of the present invention, in the absorbent article of the fifteenth aspect, while in the first mode of usage, the belt-shaped member pushes up the absorbent article main body to one side during wearing, by being extended and fixed to the target object.

According to the sixteenth aspect of the present invention, the first mode of usage for the absorbent article uses the belt-shaped member in the state where it is extended and engaged with a predetermined target object such as underwear or the wearer's buttocks. This mode of usage allows the belt-shaped member to press the absorbent article main body against a predetermined body part of the wearer and, for example, makes it possible to attain the state of being pressed against the curvature of the wearer's body. Furthermore, the stress generated in the belt-shaped member can be directed from the entirety of the absorbent article main body to a predetermined body part of the wearer, thereby allowing the entire absorbent article main body to be in contact with the predetermined body part of the wearer.

In a seventeenth aspect of the present invention, an absorbent article having a substantially elongated shape includes: an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and a belt-shaped member that is liquid permeable disposed between the top sheet portion and the absorbent core portion so as to be slidable in a longitudinal direction of the absorbent article main body, in which an engaging portion is provided in at least one end side of both ends in the longitudinal direction of the belt-shaped member.

According to a seventeenth aspect of the present invention, the absorbent article includes an absorbent article main body including at least a top sheet portion that is at least partially liquid permeable and disposed on a first side in a thickness direction and a liquid retentive absorbent core portion disposed on a second side in the thickness direction of the absorbent article, i.e. on an opposite side of the top sheet portion; and a belt-shaped member that is liquid permeable disposed between the top sheet portion and the absorbent core portion so as to be slidable in a longitudinal direction of the absorbent article main body and is formed in a substantially elongated shape.

Thus, the belt-shaped member is slidably disposed between the top sheet portion and the absorbent core portion in the absorbent article main body, and both ends of the belt-shaped member are engaged with a predetermined portion of a target object such as underwear, so that the material closer to the skin surface than the absorbent body portion, namely the top sheet, can be lifted to the wearer's body by the belt-shaped member. Therefore, even a wearer with sensitive skin is not always subjected to the stiffness of the absorbent core portion. It is also possible to prevent the leakage of menstrual blood along the skin.

According to an eighteenth aspect, in the absorbent article described in the seventeenth aspect, the belt-shaped member is formed to be at least partially extensible.

According to the eighteenth aspect of the present invention, the belt-shaped member is formed to be at least partially extensible. Thus, the extensibility of the belt-shaped member can ensure, for example, a predetermined length of the belt-shaped member necessary for attaching thereof to a target object such as underwear. In addition, since the belt-shaped member is not extended when not attached thereto, for example, it is possible to reduce the size of the belt-shaped member in the absorbent article.

According to a nineteenth aspect, in the absorbent article described in the seventeenth aspect, the belt-shaped member is formed to be at least partially stretchable.

According to the nineteenth aspect of the present invention, the belt-shaped member is formed to be at least partially stretchable. Thus, the stretchability of the belt-shaped member can keep the absorbent article main body adhered to the wearer's body since, for example, even if a relative dislocation occurs between underwear and the wearer's body due to the loosening of underwear or deformation thereof following movement of the wearer's body, the belt-shaped member can expand and contract following the dislocation. Therefore, adhesion between the wearer's excretory part and the like and the absorbent article is improved, thereby preventing leakage of excrement such as menstrual blood.

In a twentieth aspect, an absorbent article having a substantially elongated shape includes: an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and a pair of belt-shaped members, each of which is disposed on the second side of the absorbent article main body, along a longitudinal direction of the absorbent article main body, in which each of the pair of belt-shaped members joins the absorbent article main body in a predetermined position on the second side of the absorbent article; and an engaging portion is provided in at least one end side of both ends in the longitudinal direction of the each of the pair of belt-shaped members.

According to the twentieth aspect of the present invention, the absorbent article includes: an absorbent article main body including a top sheet portion that is at least partially liquid permeable and disposed on a first side in a thickness direction of the absorbent article and a liquid retentive absorbent core portion disposed on a second side in the thickness direction of the absorbent article, i.e. on an opposite side of the top sheet portion; and a pair of belt-shaped members, each of which is disposed on the second side of the absorbent article main body, along a longitudinal direction thereof, and has a substantially elongated shape. The belt-shaped members extend in the longitudinal direction, so as to be substantially parallel to each other in the width direction. Among both ends in the longitudinal direction of each belt-shaped member, an engaging portion is provided in at least one end side.

Therefore, in the absorbent article, a pair of belt-shaped members is arranged along the longitudinal direction of the absorbent article main body, and both end portions of the belt-shaped members are respectively fixed or engaged with a target object such as underwear. Thus, since each belt-shaped member is able to deform independently, for example, it becomes easy to follow the movement of wearer's buttocks.

In a twenty-first aspect of the present invention, an absorbent article having a substantially elongated shape includes: an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and a pair of belt-shaped members, each of which is disposed on the second side of the absorbent article main body, extending in an opposite direction to each other in the longitudinal direction of the absorbent article main body, in which a first end in a longitudinal direction of each of the pair of belt-shaped members joins the absorbent article main body in a predetermined position on the second side of the absorbent article; and an engaging portion is provided at a second end in the longitudinal direction of the each of the pair of belt-shaped members.

According to the twenty-first aspect of the present invention, the absorbent article includes: an absorbent article main body including a top sheet portion that is at least partially liquid permeable and disposed on a first side in a thickness direction of the absorbent article and a liquid retentive absorbent core portion disposed on a second side in the thickness direction of the absorbent article, i.e. on an opposite side of the top sheet portion and a pair of belt-shaped members, each of which is disposed on the second side of the absorbent article main body, extending in an opposite direction to each other in the longitudinal direction of the absorbent article main body, and has a substantially elongated shape. The first end in a longitudinal direction of each belt-shaped member joins the absorbent article main body in a predetermined position on the second side of the absorbent article. In addition, an engaging portion is created in the second end in the longitudinal direction of each belt-shaped member.

Therefore, in the absorbent article, a belt-shaped member divided into two pieces is arranged along the longitudinal direction of the absorbent article main body, and both end portions of the belt-shaped member are respectively fixed or engaged with a target object such as underwear. This allows each belt-shaped member to lift the entire absorbent article main body toward the wearer's body.

### Effects of the Invention

Accordingly, the present invention can provide an absorbent article capable of preventing the leakage of excrement such as body fluid, by adhering to the excretory part of the wearer without dislocation.

### BRIEF DESCRIPTION OF THE DRAWINGS

FIG. 1 is a front view of a sanitary napkin according to a first embodiment of the present invention;
FIG. 2 is a back view of FIG. 1;
FIG. 3 is a sectional view taken along the line A-A of FIG. 1;
FIG. 4A is a sectional view taken along the line B-B of FIG. 1;
FIG. 4B is a sectional view taken along the line C-C of FIG. 1;
FIG. 4C is a sectional view taken along the line D-D of FIG. 1;
FIG. 5 is a front view of a belt-shaped member according to the first embodiment;
FIG. 6A is a diagram showing another embodiment of the belt-shaped member;
FIG. 6B is a diagram showing another embodiment of the belt-shaped member;
FIG. 7A is a partially enlarged view of FIG. 6A;
FIG. 7B is a diagram showing still another embodiment of FIG. 7A;
FIG. 8 is a front view showing a configuration when attaching the sanitary napkin according to the first embodiment;
FIG. 9A is a diagram showing the attached state of the sanitary napkin according to the first embodiment;
FIG. 9B is a diagram showing the attached state of the sanitary napkin according to the first embodiment;
FIG. 9C is a diagram showing the attached state of the sanitary napkin according to the first embodiment;
FIG. 10 is a front view of a sanitary napkin according to a second embodiment of the present invention;
FIG. 11 is a back view of FIG. 10;
FIG. 12 is a sectional view taken along the line V-V of FIG. 10;
FIG. 13A is a sectional view taken along the line X-X of FIG. 10;
FIG. 13B is a sectional view taken along the line Y-Y of FIG. 10;
FIG. 13C is a sectional view taken along the line Z-Z of FIG. 10;
FIG. 14 is a sectional view taken along the line V-V, showing a modification of the sanitary napkin according to the second embodiment;
FIG. 15 is a back view of a sanitary napkin according to a third embodiment of the present invention;
FIG. 16 is a back view of FIG. 15;
FIG. 17 is a sectional view taken along the line G-G of FIG. 15;
FIG. 18A is a sectional view taken along the line H-H of FIG. 17;
FIG. 18B is a sectional view taken along the line I-I of FIG. 17;
FIG. 18C is a sectional view taken along the line J-J of FIG. 17;
FIG. 19A is a front view illustrating a main body of a stretchable sanitary napkin;
FIG. 19B is a front view illustrating a main body of a stretchable sanitary napkin;
FIG. 20A is a front view illustrating a main body of a stretchable sanitary napkin;
FIG. 20B is a front view illustrating a main body of a stretchable sanitary napkin;
FIG. 21 is a back view of a sanitary napkin according to a fourth embodiment of the present invention;
FIG. 22 is a sectional view taken along the line K-K in FIG. 21;
FIG. 23A is a sectional view taken along the line L-L of FIG. 21;
FIG. 23B is a sectional view taken along the line M-M of FIG. 21;
FIG. 23C is a sectional view taken along the line N-N of FIG. 21;
FIG. 24 is a back view of a sanitary napkin according to a fifth embodiment of the present invention;
FIG. 25A is a sectional view taken along the line O-O of FIG. 24;
FIG. 25B is a diagram showing a modification of FIG. 24;
FIG. 26A is a front view of a sanitary napkin according to a sixth embodiment of the present invention;
FIG. 26B is a sectional view taken along the line P-P of FIG. 26A;
FIG. 27 is a diagram showing another embodiment of FIG. 21;
FIG. 28 is a front view of a sanitary napkin according to a seventh embodiment of the present invention;
FIG. 29A is a sectional view taken along the line Q-Q of FIG. 28;
FIG. 29B is a sectional view taken along the line R-R of FIG. 28;
FIG. 30 is a back view of a sanitary napkin according to an eighth embodiment of the present invention;
FIG. 31A is a sectional view taken along the line S-S of FIG. 30;
FIG. 31B is a modification of FIG. 30;
FIG. 32A is a back view of a sanitary napkin according to a ninth embodiment of the present invention;
FIG. 32B is a back view of a sanitary napkin according to the ninth embodiment of the present invention;
FIG. 33 is a back view of a sanitary napkin according to a tenth embodiment of the present invention;
FIG. 34A is a back view of a sanitary napkin according to an eleventh embodiment of the present invention;
FIG. 34B is a back view of a sanitary napkin according to the eleventh embodiment of the present invention;
FIG. 35A is a front view of a sanitary napkin according to a twelfth embodiment of the present invention;
FIG. 35B is a sectional view taken along the line T-T of FIG. 35A;
FIG. 36 is a back view of a sanitary napkin according to a thirteenth embodiment of the present invention;
FIG. 37 is a back view of a sanitary napkin according to a fourteenth embodiment of the present invention;
FIG. 38 is a back view of a sanitary napkin according to a fifteenth embodiment of the present invention;
FIG. 39 is a back view of a sanitary napkin according to a sixteenth embodiment of the present invention;
FIG. 40 is a back view of a sanitary napkin according to a seventeenth embodiment of the present invention; and
FIG. 41 is a sectional view taken along the line U-U of FIG. 40.

### PREFERRED MODE FOR CARRYING OUT THE INVENTION

Embodiments of the present invention are described hereinafter with reference to the accompanying drawings. Furthermore, it is to be understood that the embodiments of the present invention are not limited thereto, and the technical scope of the present invention is not limited thereto.

In addition, although the absorbent articles according to the present invention are positioned on the crotch of the wearer's body in order to absorb menstrual blood, urine, and leukorrhea discharged from the wearer's body, the following preferred embodiments are directed to sanitary napkins, the primary object of which is to absorb menstrual blood discharged from the vaginal opening of females. Moreover, in the following description, one of two surfaces of the absorbent article, which is directed to the excretory part, is called "skin contacting side", and the other is called "skin noncontacting side", irrespective of whether or not clothing is worn on the outside thereof.

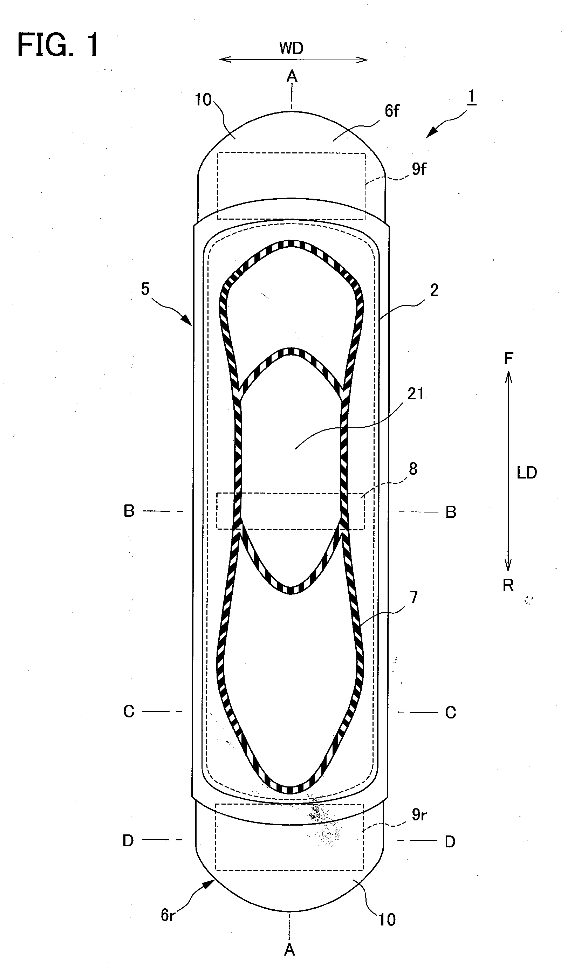

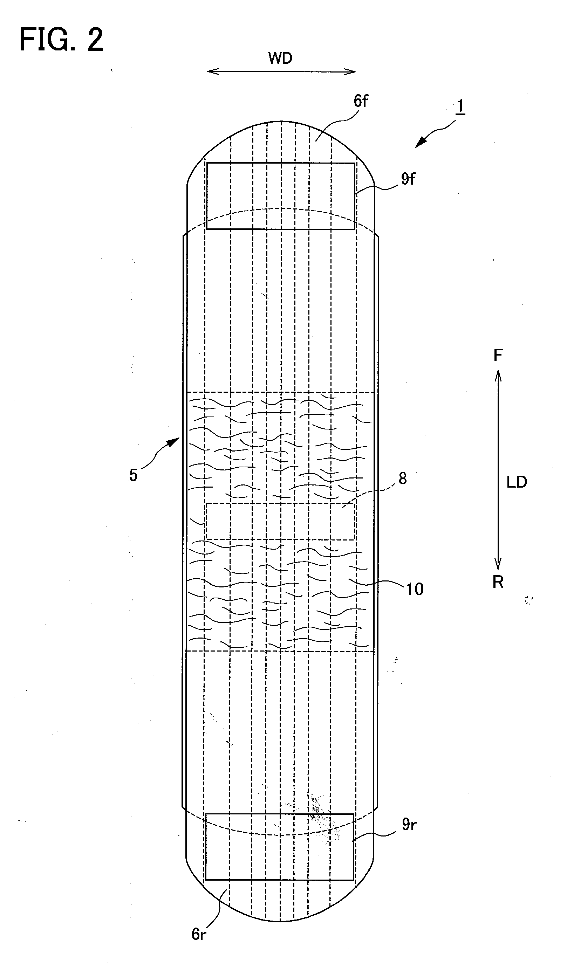

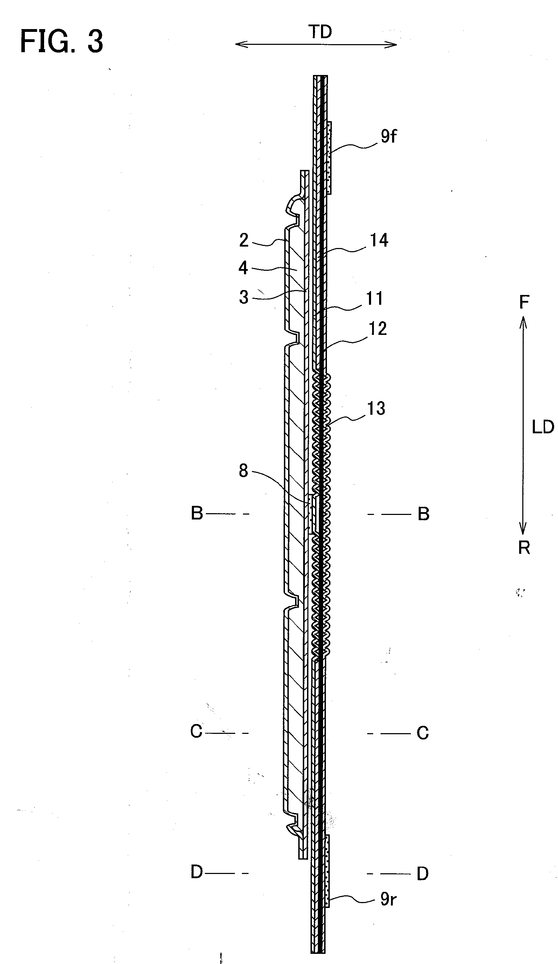

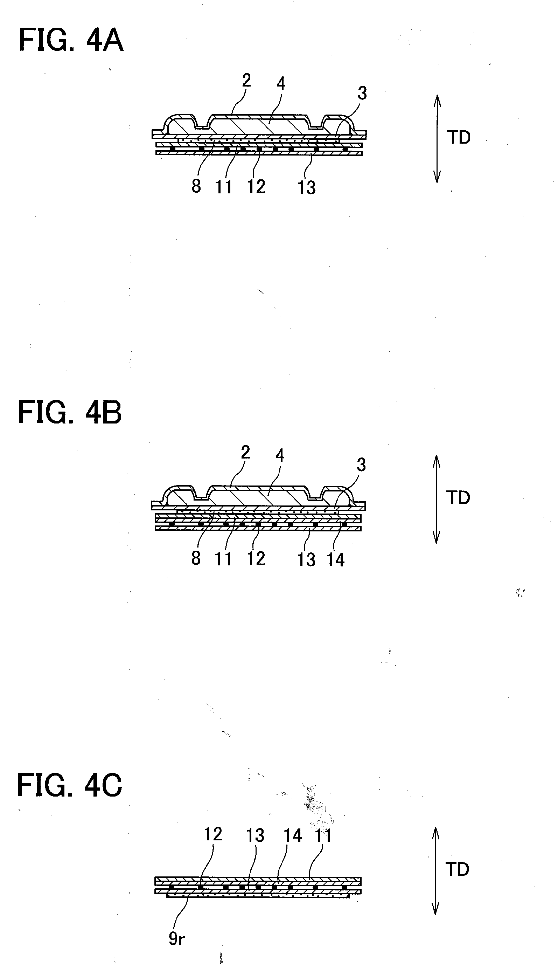

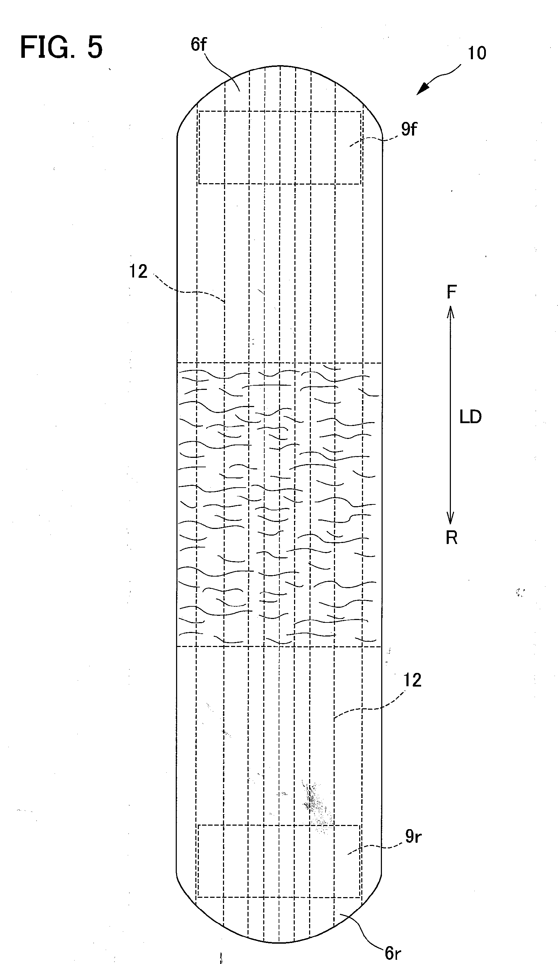

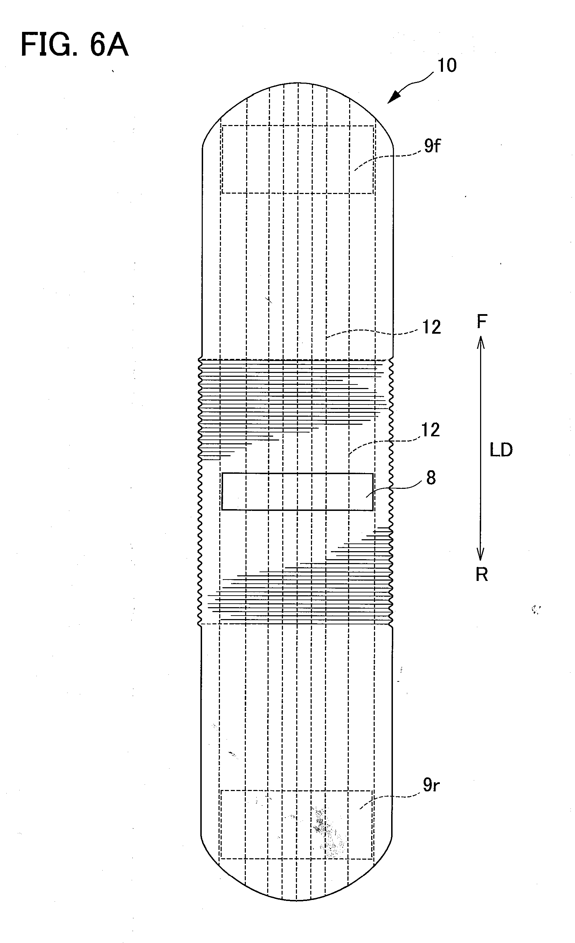

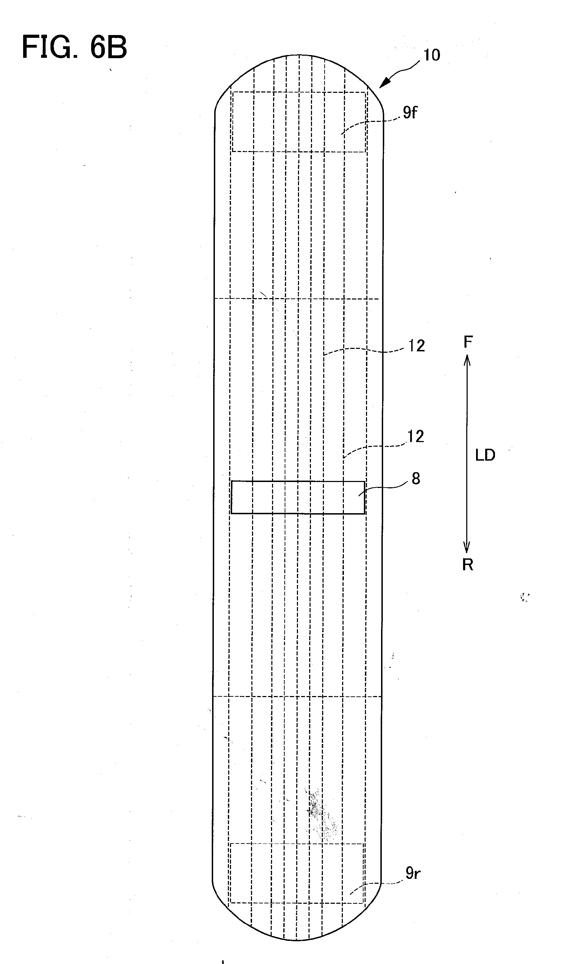

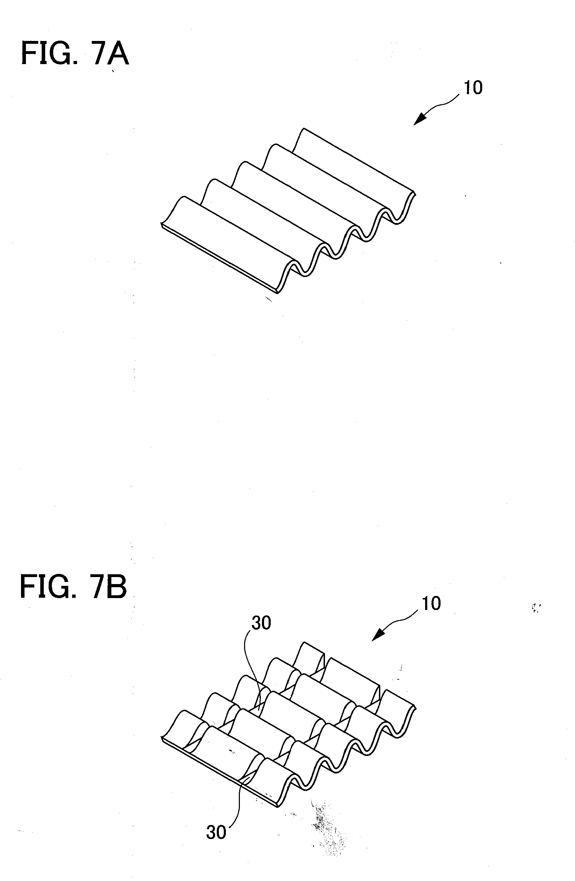

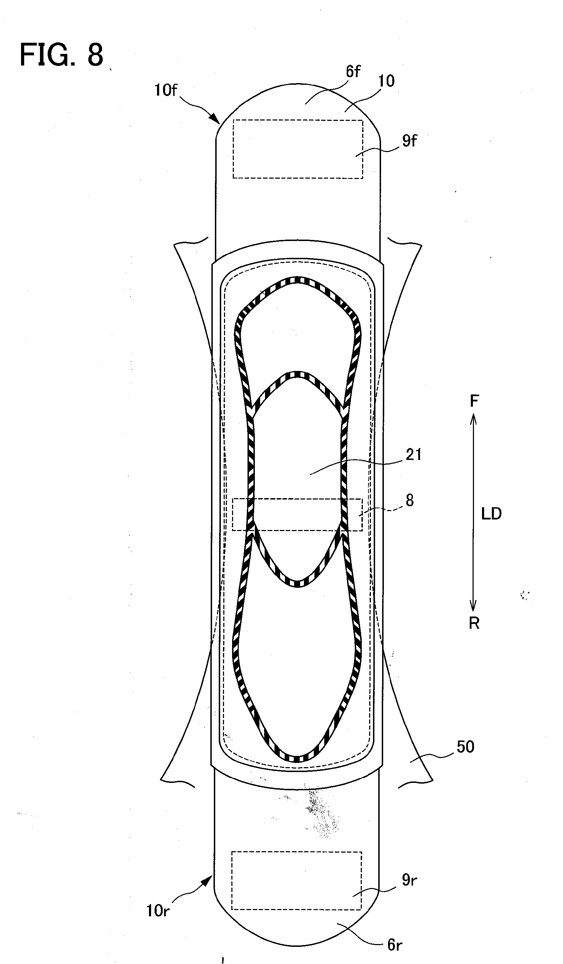

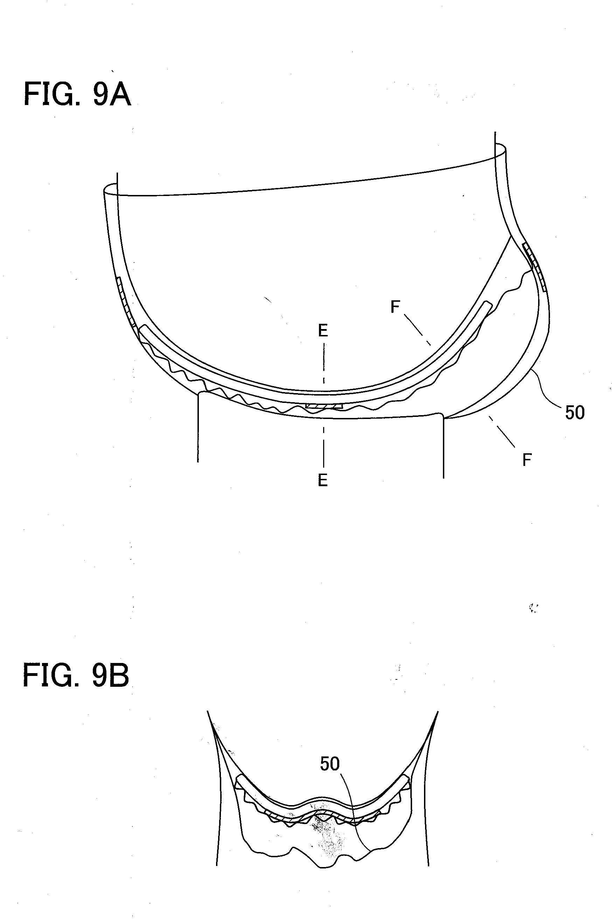

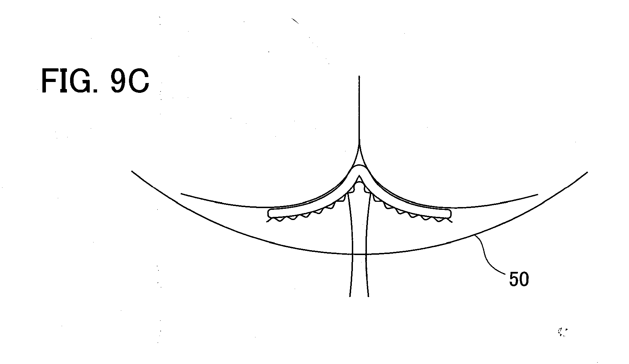

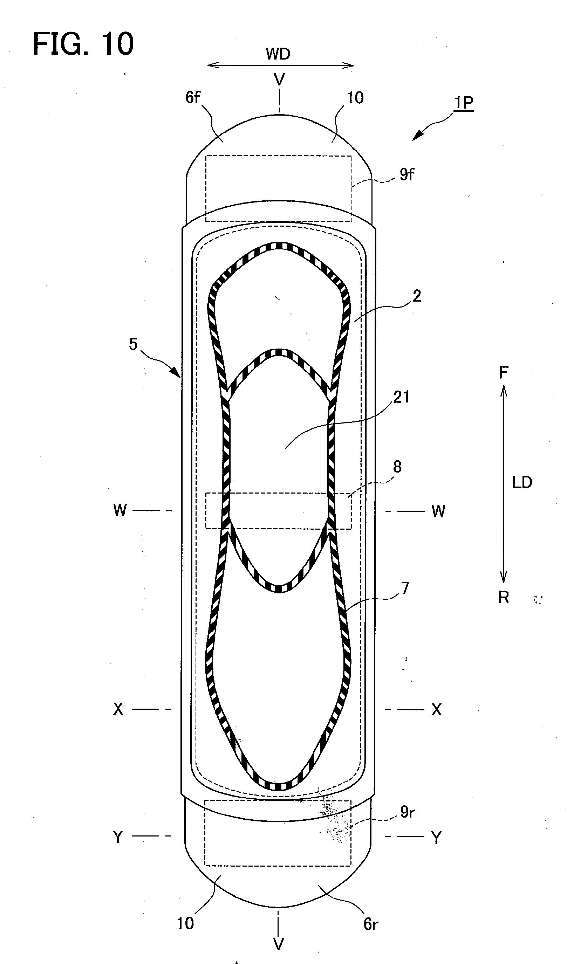

FIG. 1 is a front view of a sanitary napkin according to a first embodiment of the present invention. FIG. 2 is a back view of FIG. 1. FIG. 3 is a sectional view taken along the line A-A of FIG. 1. FIG. 4A is a sectional view taken along the line B-B of FIG. 1. FIG. 4B is a sectional view taken along the line C-C of FIG. 1. FIG. 4C is a sectional view taken along the line D-D of FIG. 1. FIG. 5 is a front view of the belt-shaped member according to the first embodiment. FIGS. 6A and 6B are diagrams showing another embodiment of the belt-shaped member. FIG. 7A is a partially enlarged view of FIG. 6A. FIG. 7B is a diagram showing another embodiment of FIG. 7A. FIG. 8 is a front view showing the configuration when attaching the sanitary napkin according to the first embodiment. FIGS. 9A, 9B and 9C are diagrams showing the attached state of the sanitary napkin according to the first embodiment. FIG. 10 is a front view of a sanitary napkin according to a second embodiment of the present invention.

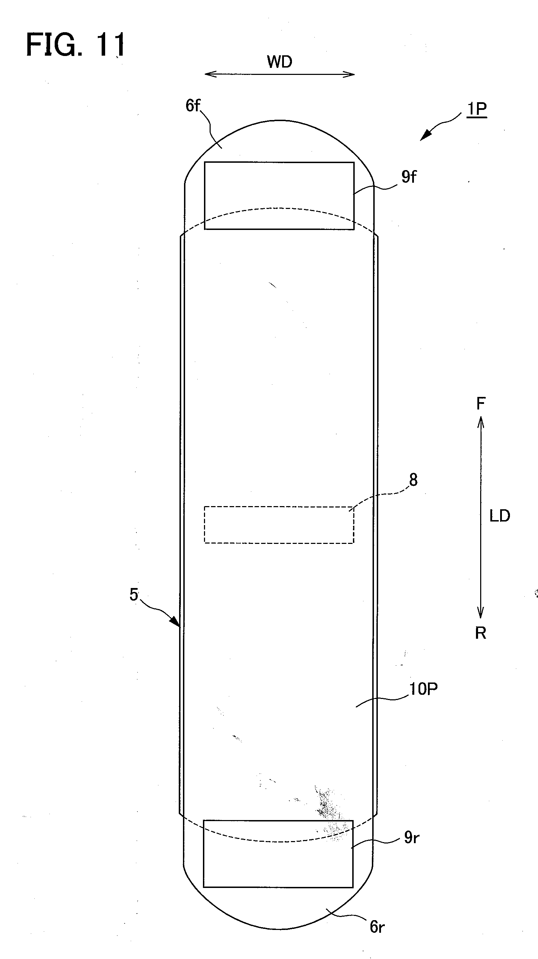

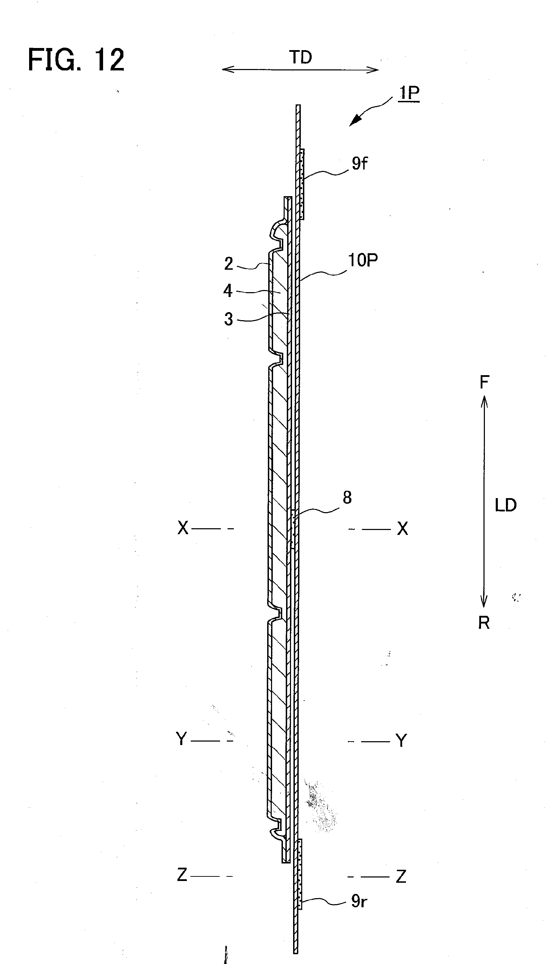

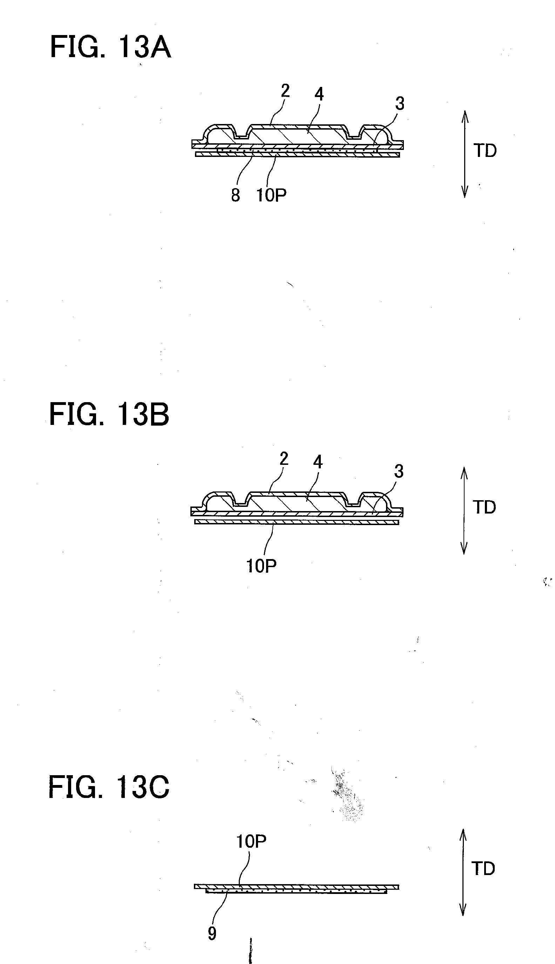

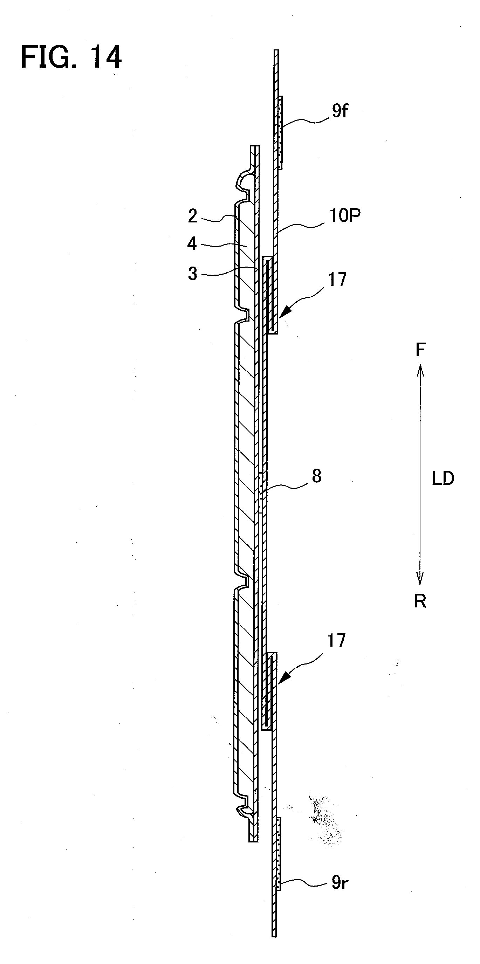

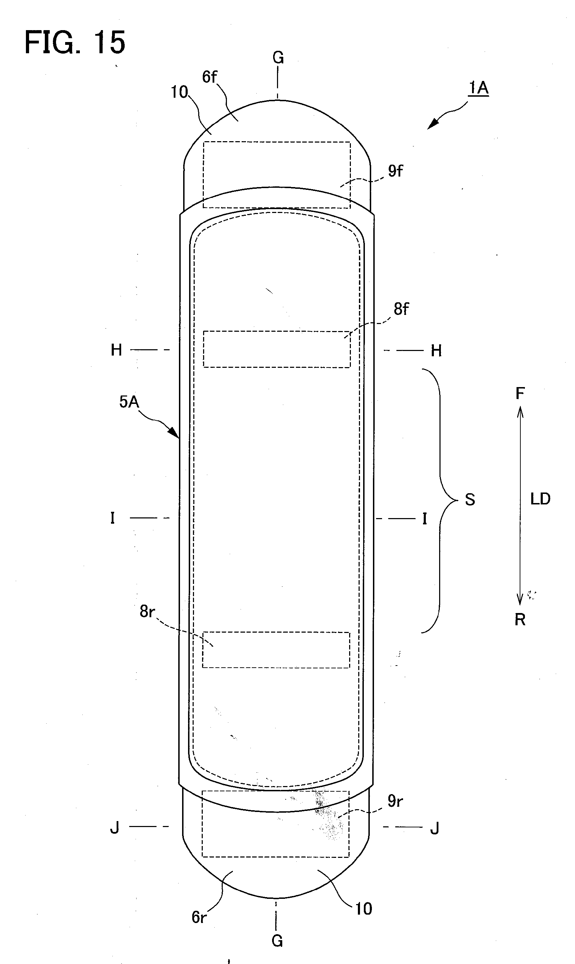

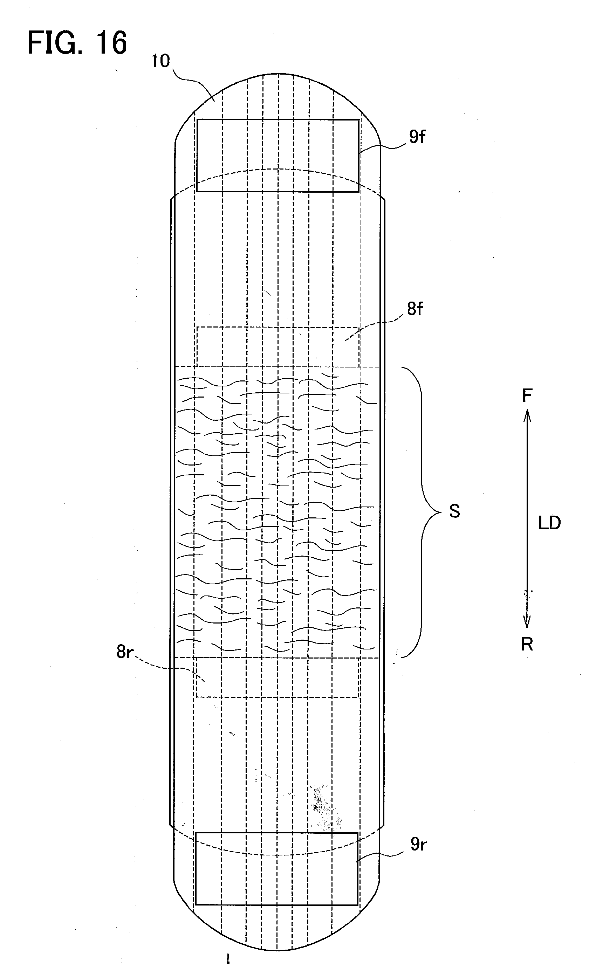

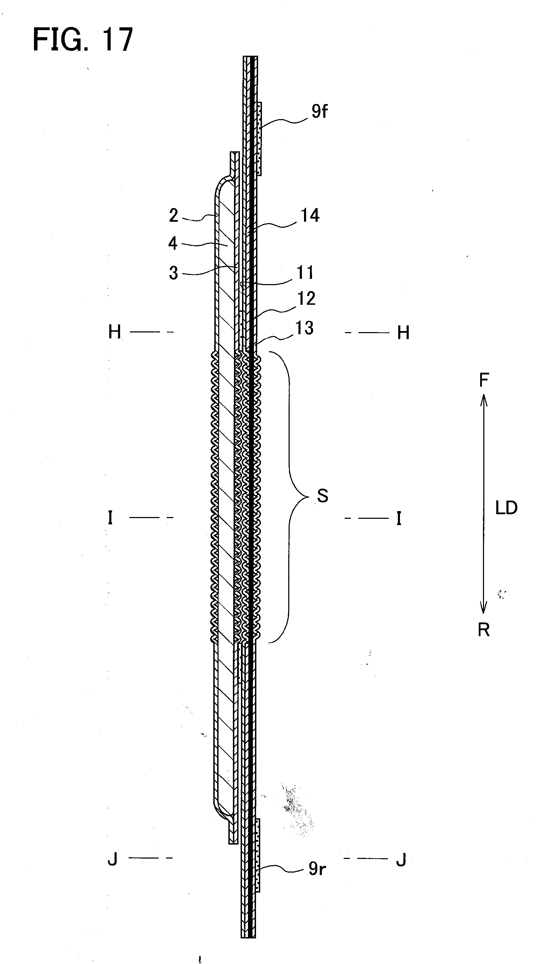

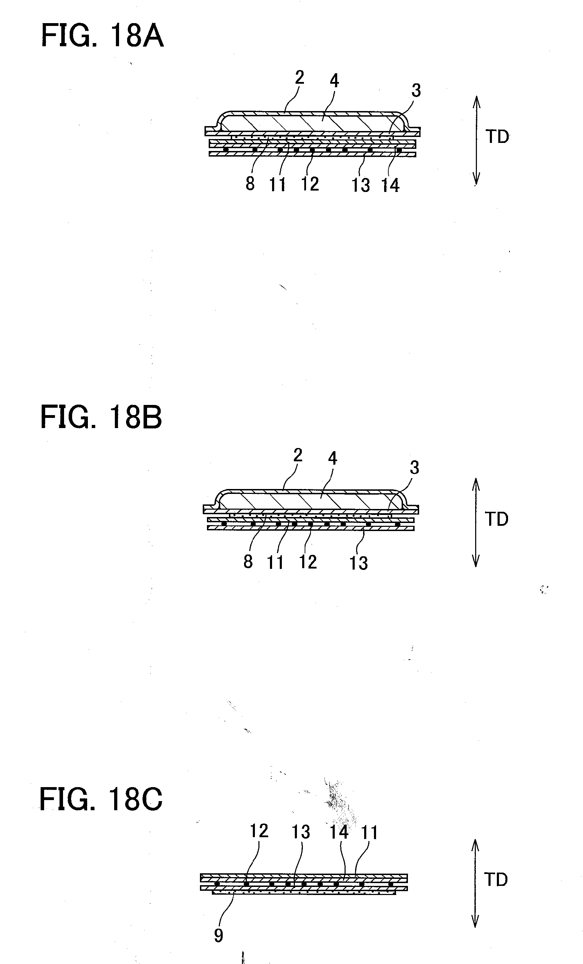

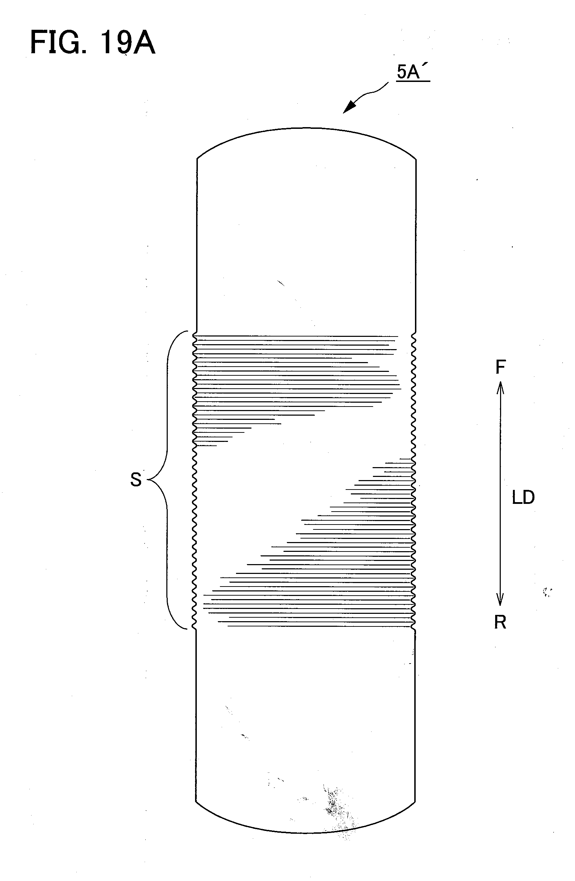

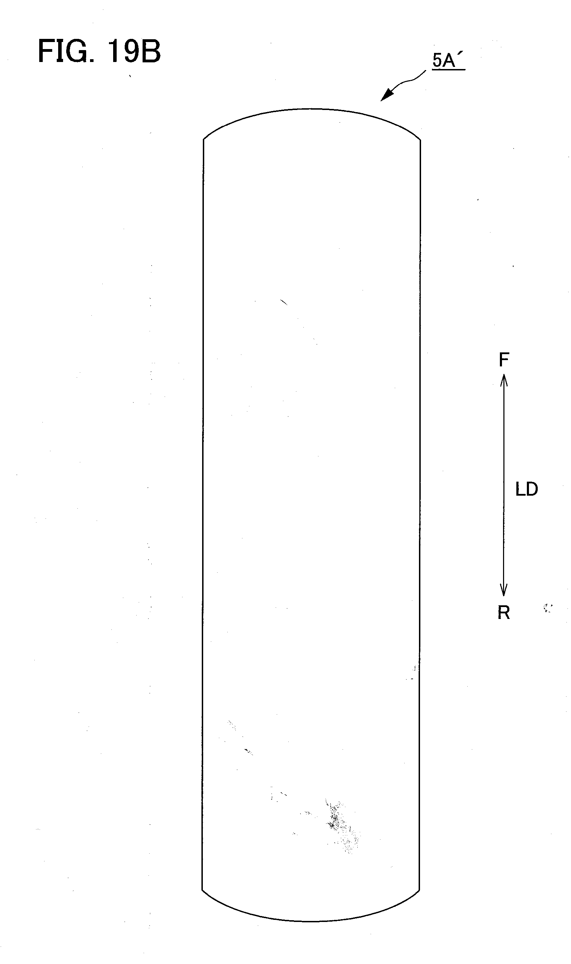

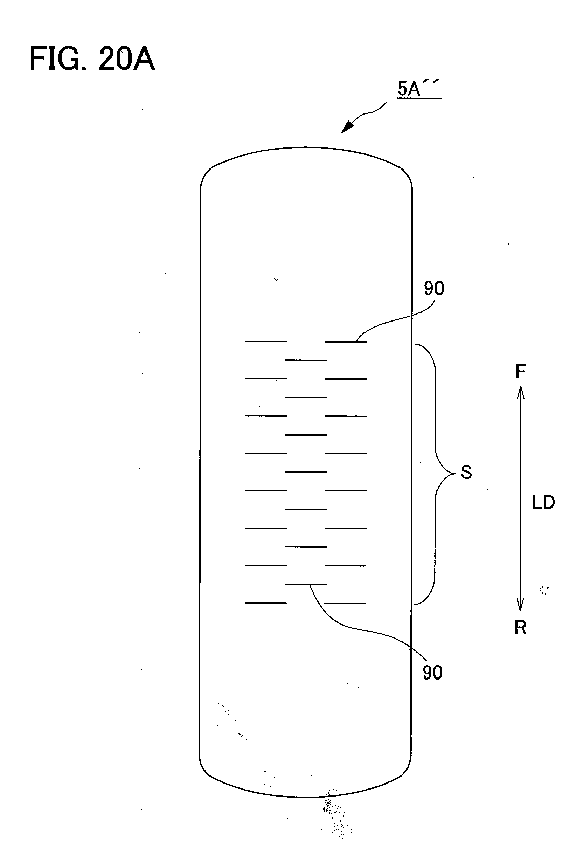

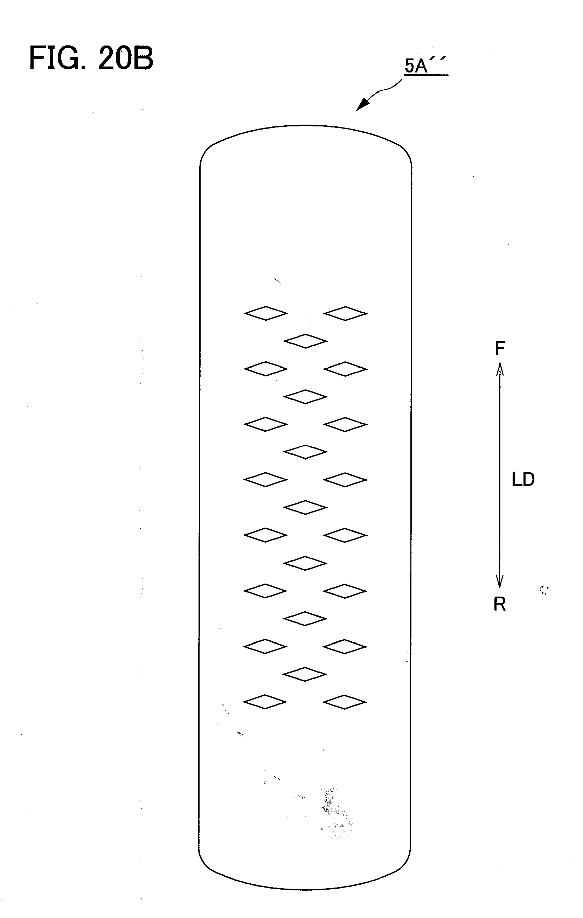

FIG. 11 is a back view of FIG. 10. FIG. 12 is a sectional view taken along the line V-V of FIG. 10. FIG. 13A is a sectional view taken along the line X-X of FIG. 10. FIG. 13B is a sectional view taken along the line Y-Y of FIG. 10. FIG. 13C is a sectional view taken along the line Z-Z of FIG. 10. FIG. 14 is a sectional view taken along the line V-V, showing a modification of the sanitary napkin according to the second embodiment. FIG. 15 is a front view of a sanitary napkin according to a third embodiment of the present invention. FIG. 16 is a back view of FIG. 15. FIG. 17 is a sectional view taken along the line G-G of FIG. 15. FIG. 18A is a sectional view taken along the line H-H of FIG. 15. FIG. 18B is a sectional view taken along the line I-I of FIG. 15. FIG. 18C is a sectional view taken along the line J-J of FIG. 15. FIGS. 19A and 19B are front views illustrating a main body of a stretchable sanitary napkin. FIGS. 20A and 20B are front views illustrating a main body of a stretchable sanitary napkin.

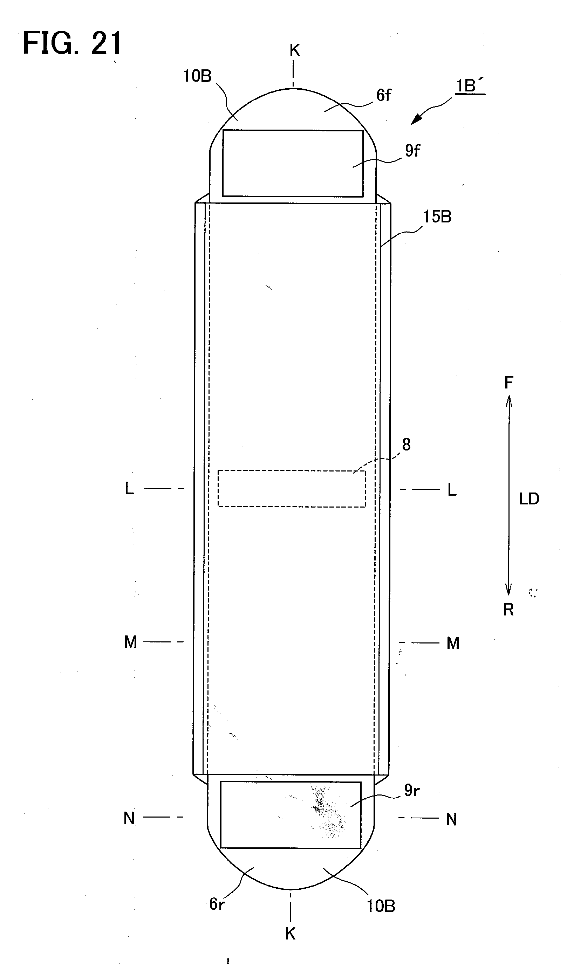

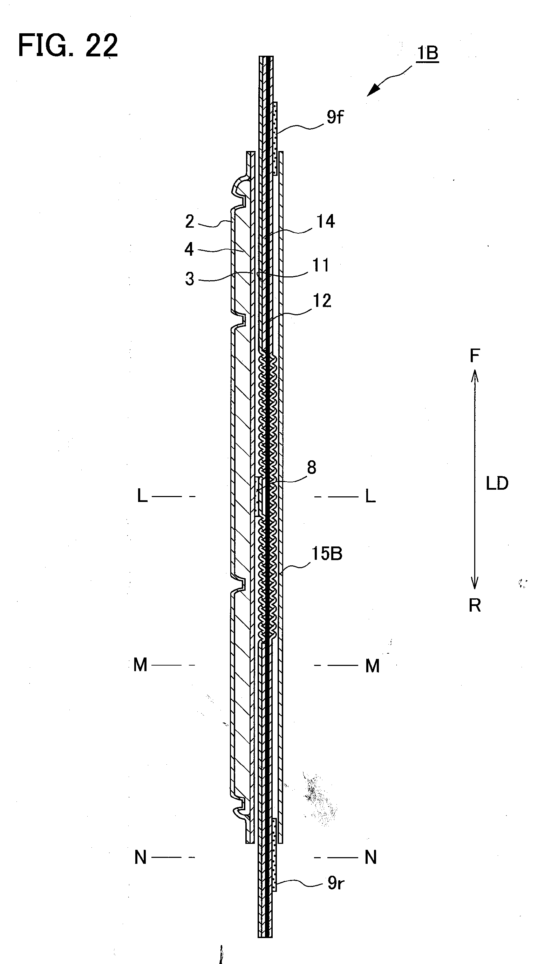

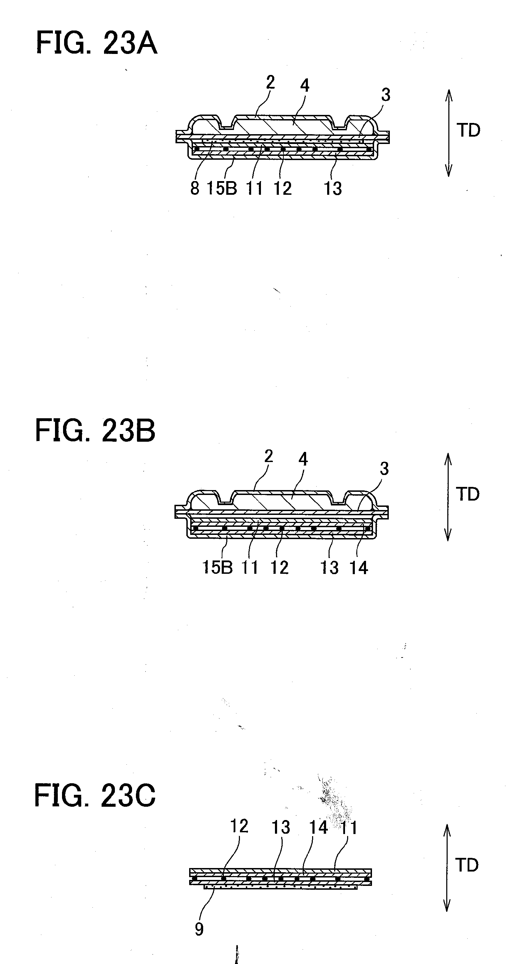

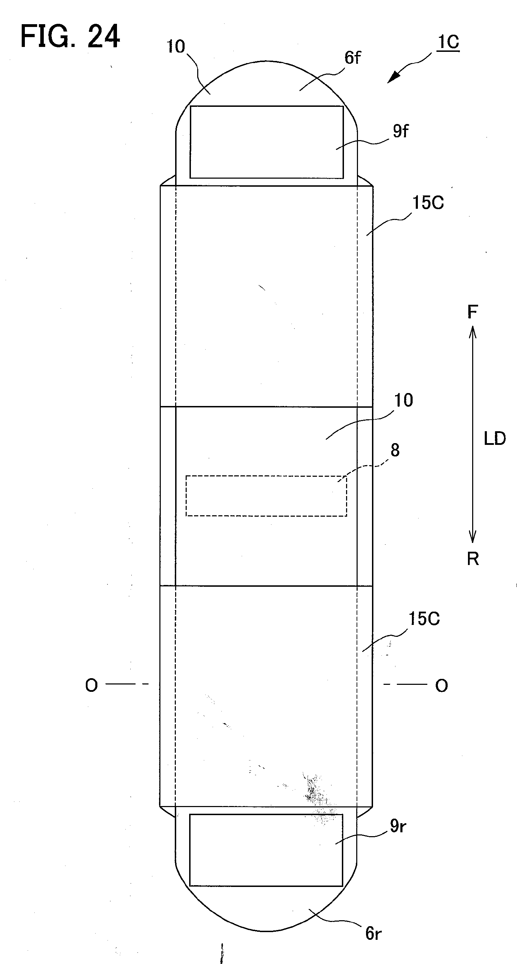

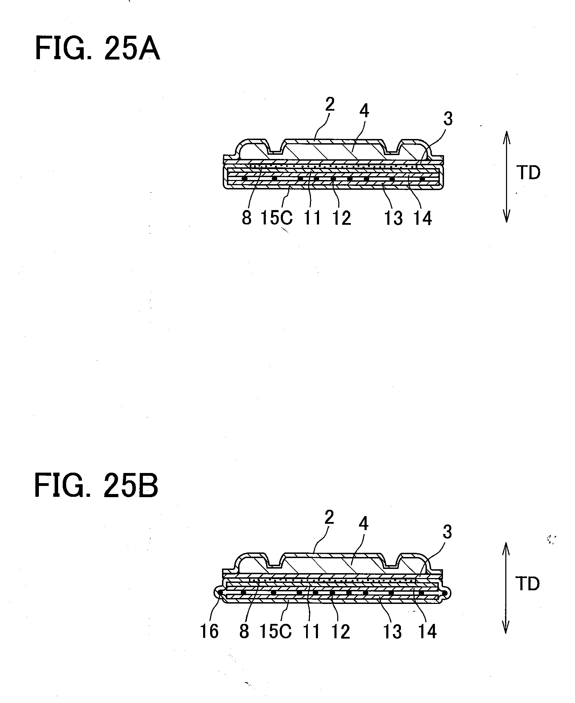

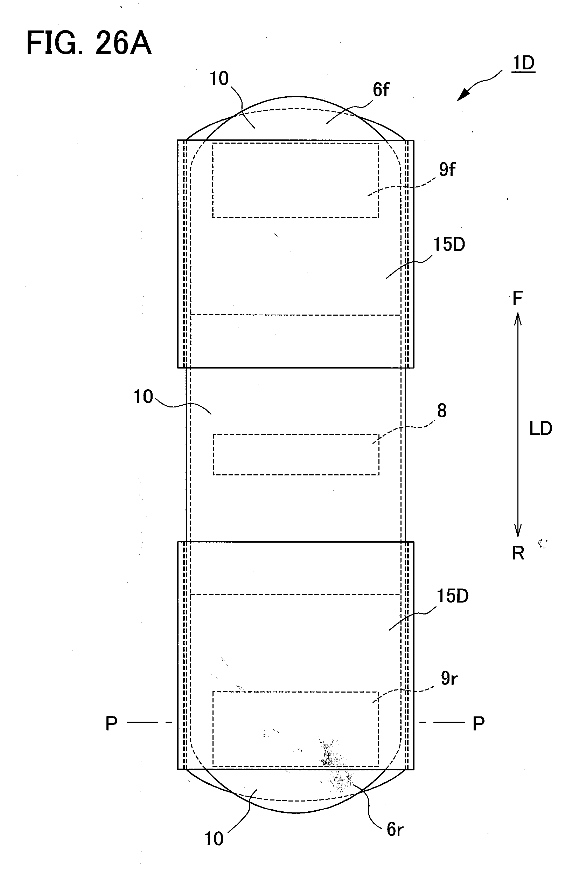

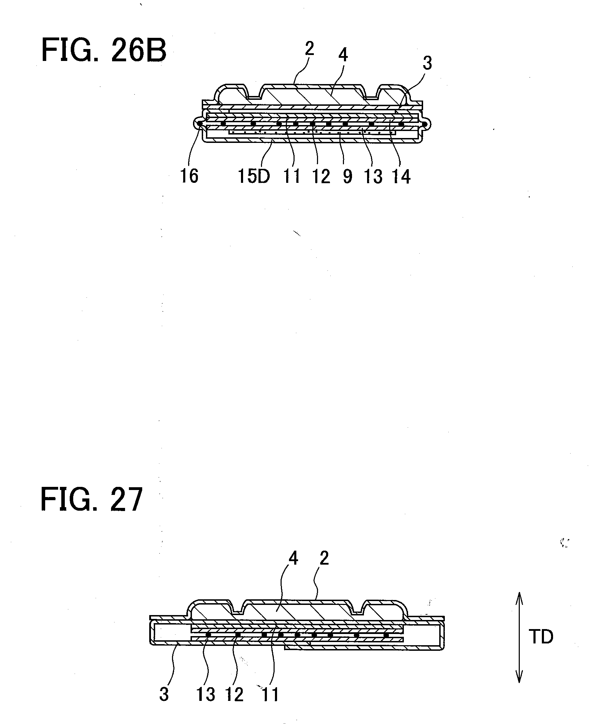

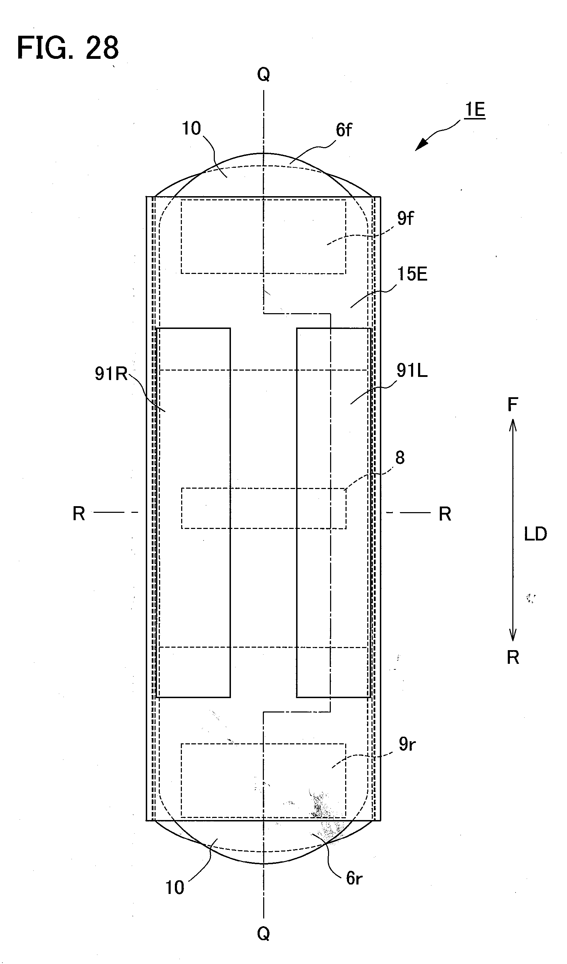

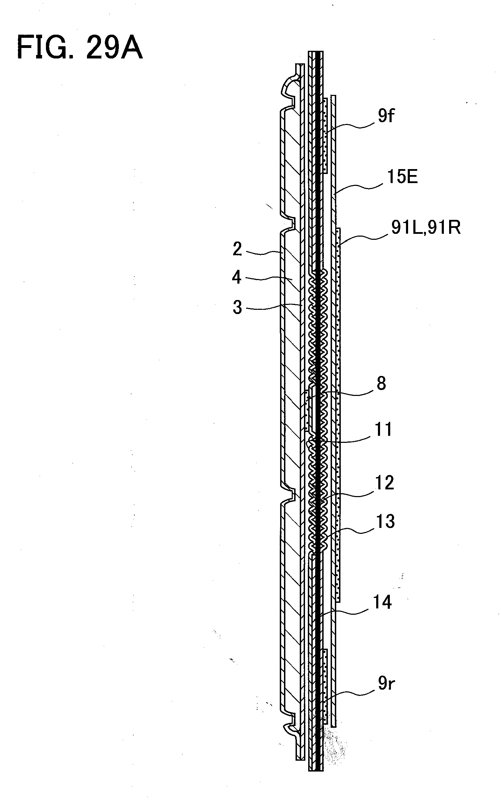

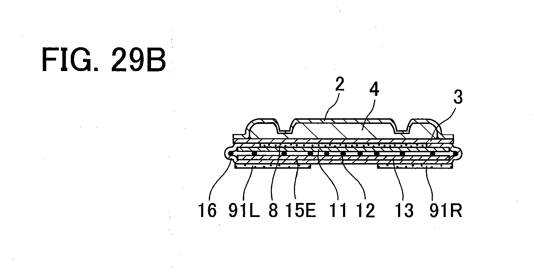

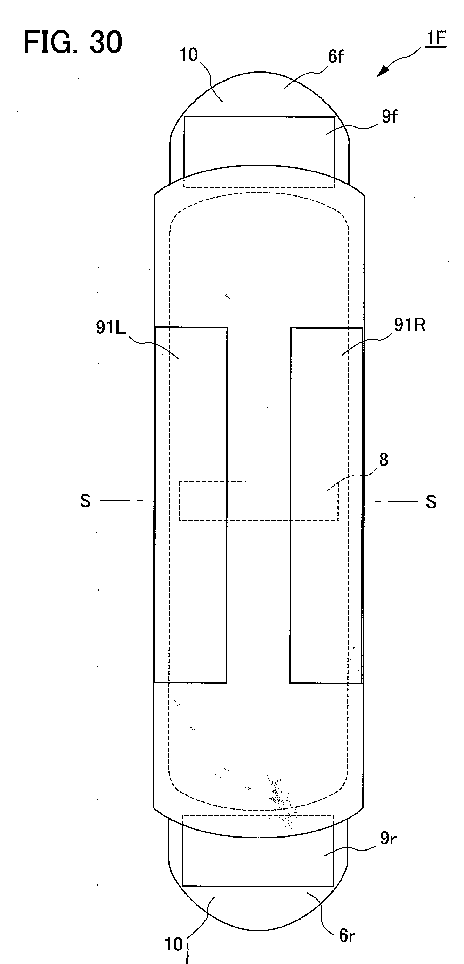

FIG. 21 is a back view of a sanitary napkin according to a fourth embodiment of the present invention. FIG. 22 is a sectional view taken along the line K-K in FIG. 21. FIG. 23A is a sectional view taken along the line L-L of FIG. 21. FIG. 23B is a sectional view taken along the line M-M of FIG. 21. FIG. 23C is a sectional view taken along the line N-N of FIG. 21. FIG. 24 is a back view of a sanitary napkin according to a fifth embodiment of the present invention. FIG. 25A is a sectional view taken along the line O-O of FIG. 24. FIG. 25B is a diagram showing a modification of FIG. 24. FIG. 26A is a front view of a sanitary napkin according to a sixth embodiment of the present invention. FIG. 26B is a sectional view taken along the line P-P of FIG. 26A. FIG. 27 is a diagram showing another embodiment of FIG. 21. FIG. 28 is a front view of a sanitary napkin according to a seventh embodiment of the present invention. FIG. 29A is a sectional view taken along the line Q-Q of FIG. 28. FIG. 29B is a sectional view taken along the line R-R of FIG. 28. FIG. 30 is a back view of a sanitary napkin according to an eighth embodiment of the present invention.

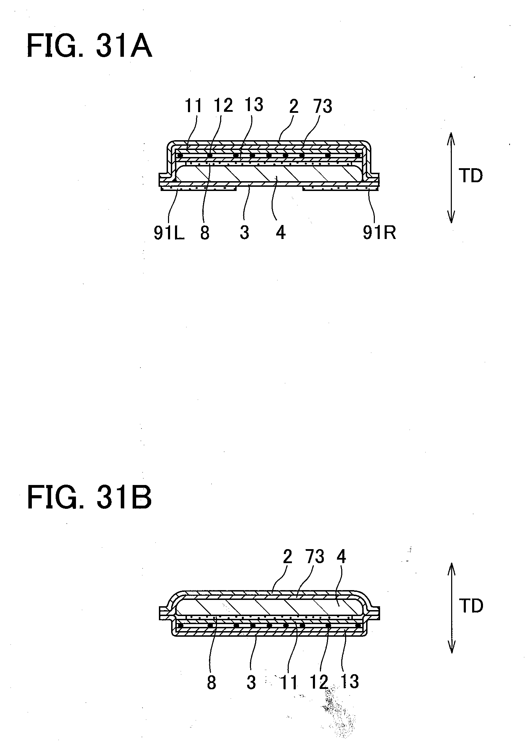

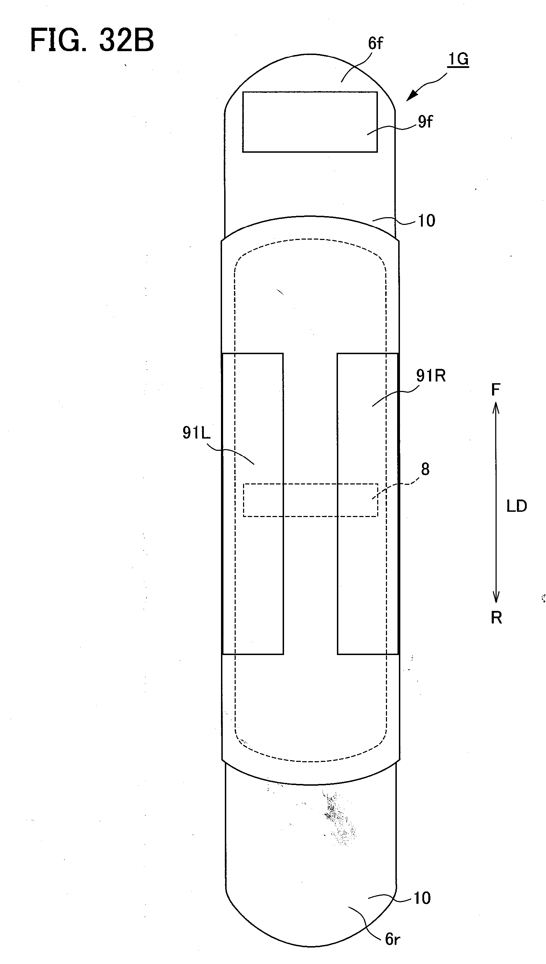

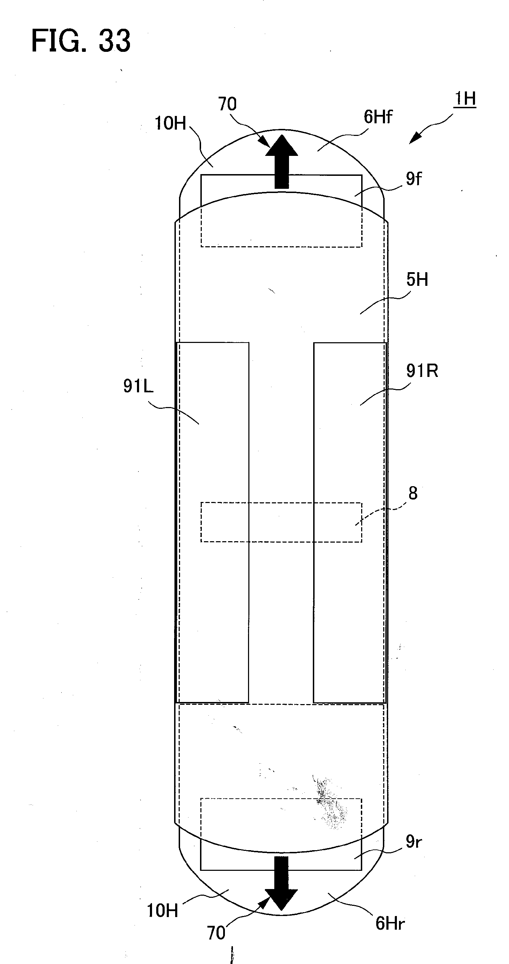

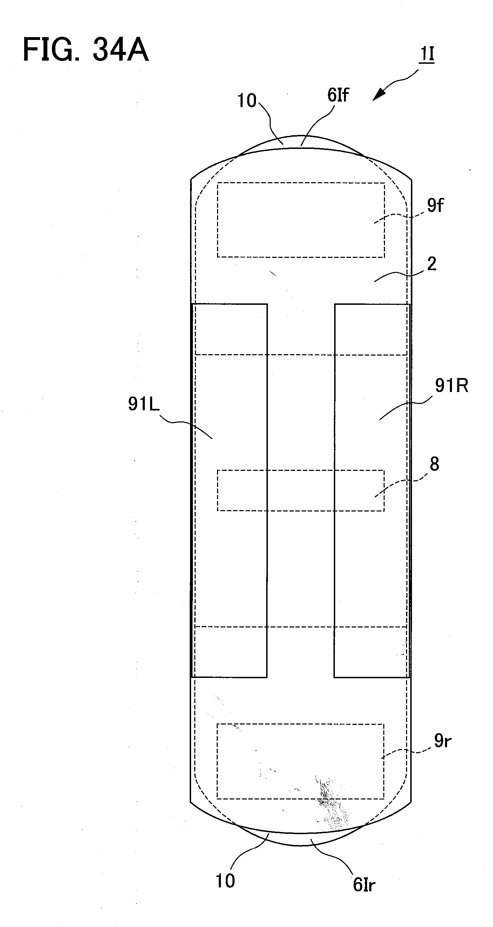

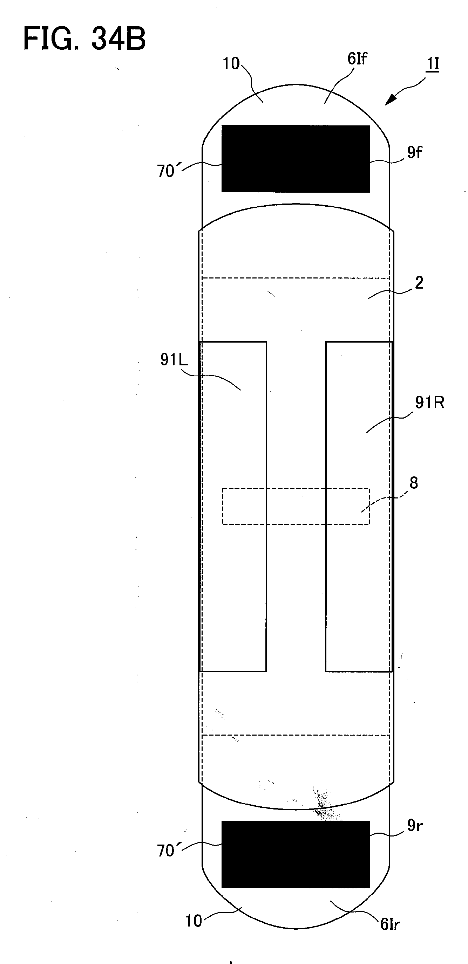

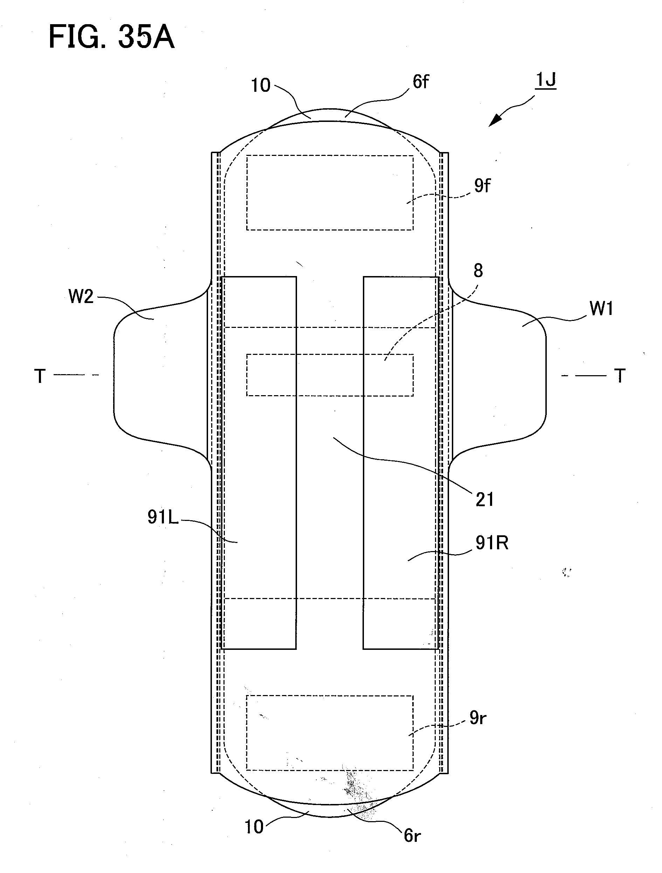

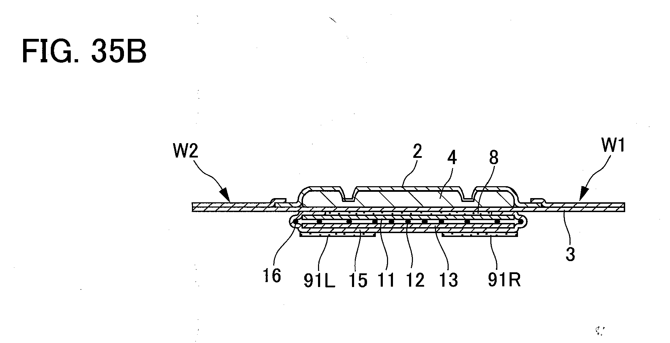

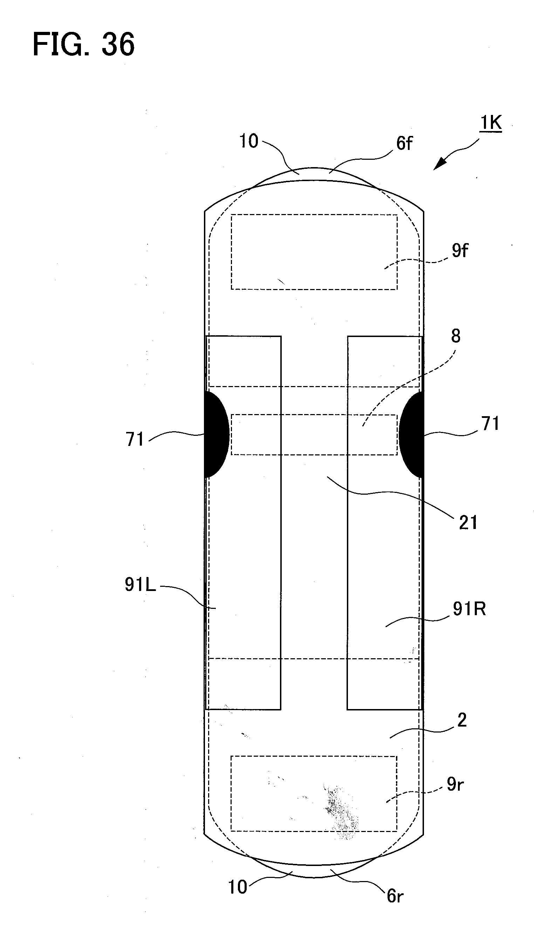

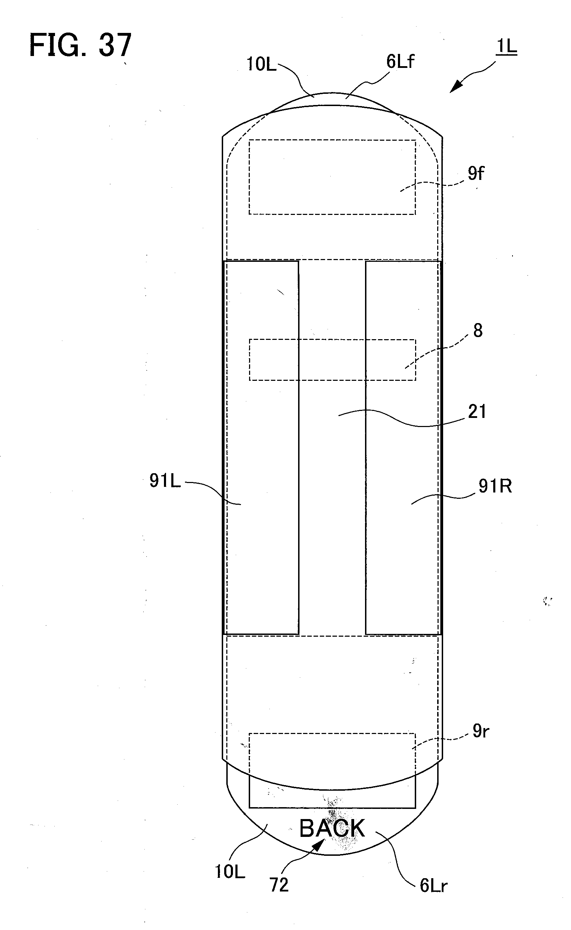

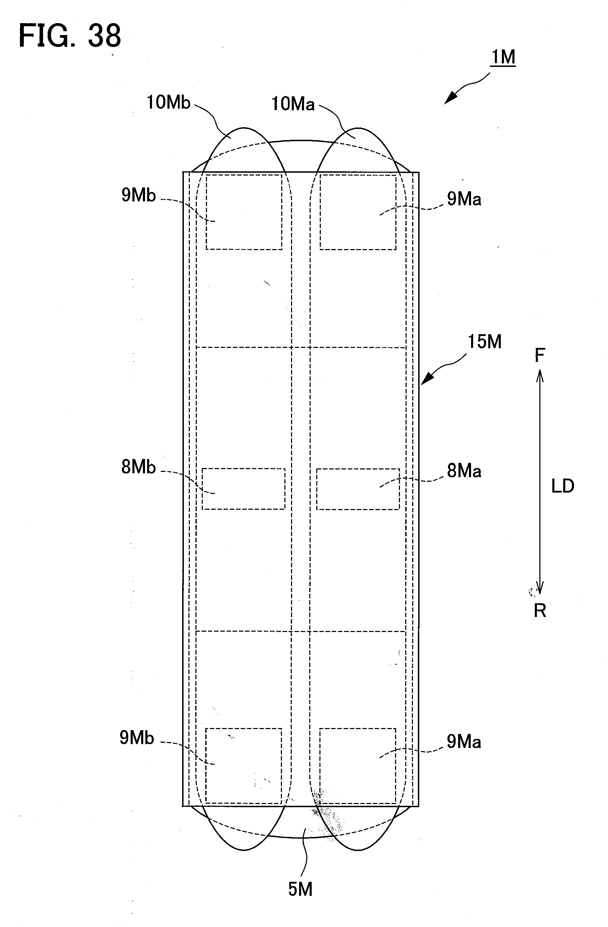

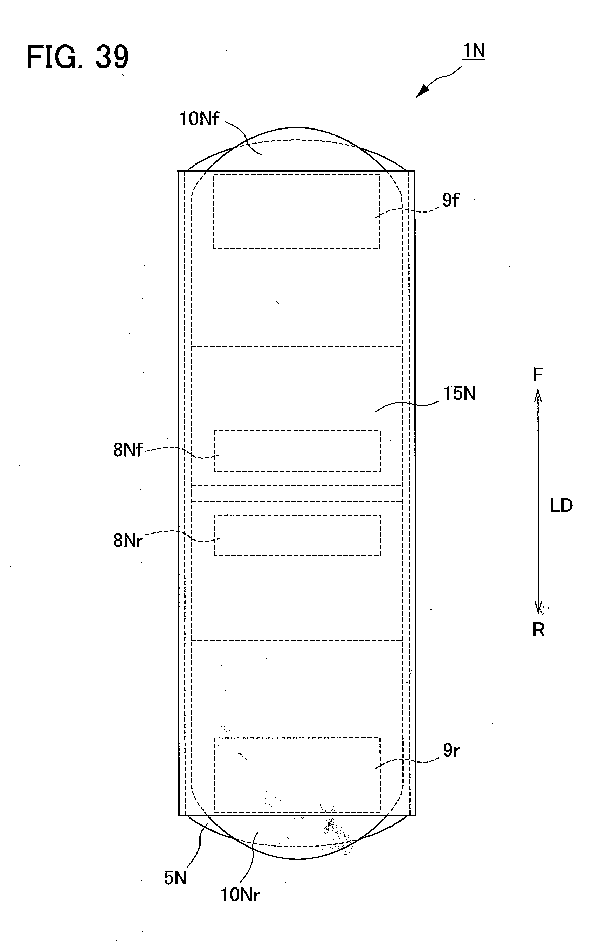

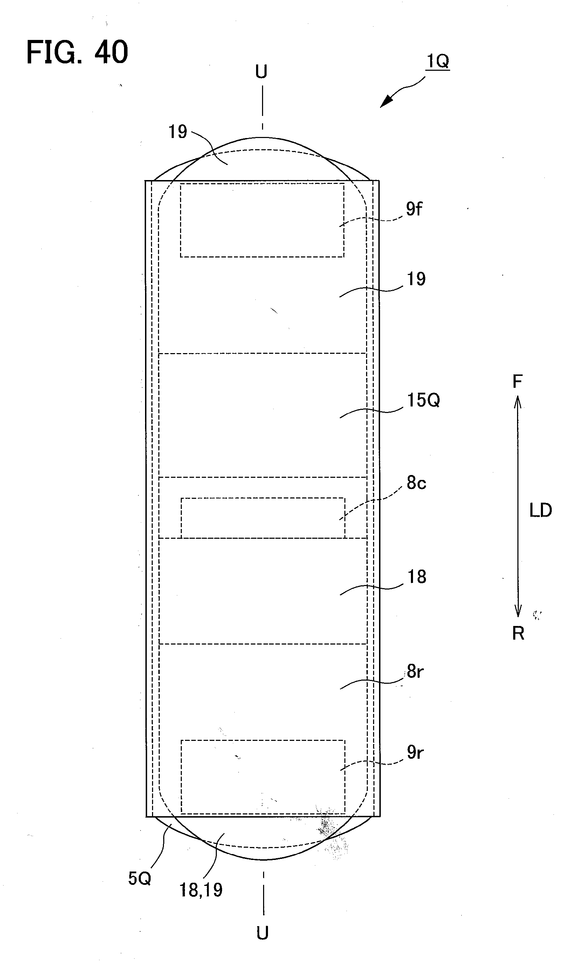

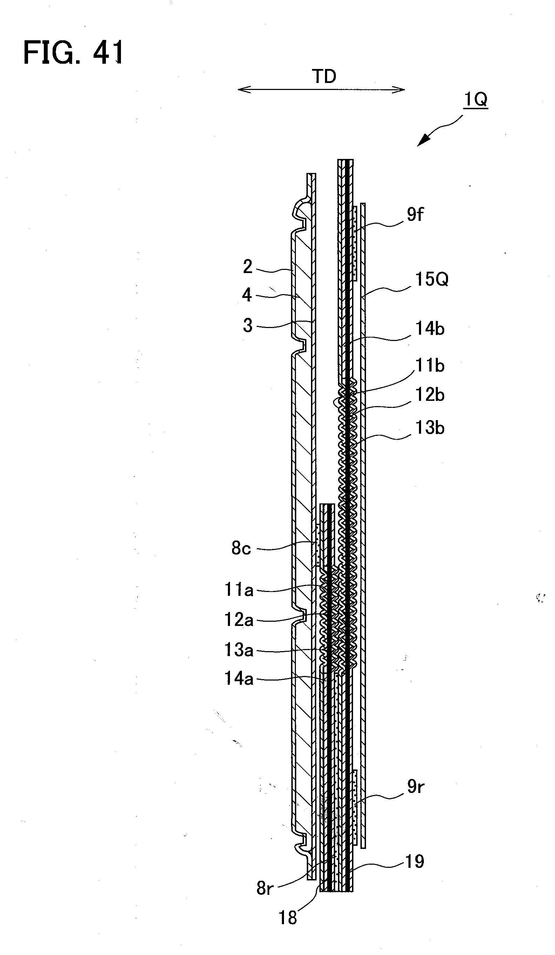

FIG. 31A is a sectional view taken along the line S-S of FIG. 30. FIG. 31B is a modification of FIG. 30. FIGS. 31A and 32B are back views of a sanitary napkin according to the ninth embodiment of the present invention. FIG. 33 is a back view of a sanitary napkin according to a tenth embodiment of the present invention. FIGS. 34A and 34B are back views of a sanitary napkin according to the eleventh embodiment of the present invention. FIG. 35A is a front view of a sanitary napkin according to a twelfth embodiment of the present invention. FIG. 35B is a sectional view taken along the line T-T of FIG. 35A. FIG. 36 is a back view of a sanitary napkin according to a thirteenth embodiment of the present invention. FIG. 37 is a back view of a sanitary napkin according to a fourteenth embodiment of the present invention. FIG. 38 is a back view of a sanitary napkin according to a fifteenth embodiment of the present invention. FIG. 39 is a back view of a sanitary napkin according to a sixteenth embodiment of the present invention. FIG. 40 is a back view of a sanitary napkin according to a seventeenth embodiment of the present invention. FIG. 41 is a sectional view taken along the line U-U of FIG. 40.

### 1. First Embodiment

### 1-1. Overall Configuration

The overall configuration of the absorbent article of the present invention is described hereinafter based on a sanitary napkin 1 according to a first embodiment of the present invention. The sanitary napkin 1 of the first embodiment has a sanitary napkin main body 5 that is an absorbent article main body, and a belt-shaped member 10. More specifically, as shown in FIGS. 1 to 4C, the sanitary napkin 1 has the sanitary napkin main body 5 and the belt-shaped member 10 that is liquid permeable. The sanitary napkin main body 5 has a liquid permeable top sheet portion 2 that constitutes a surface layer and is disposed on the skin contact side of a wearer, a liquid impermeable back sheet portion 3 that constitutes a back layer and is disposed on the skin noncontacting side of the wearer, and a liquid retentive absorbent core portion 4 that constitutes an absorption layer and is interposed between the top sheet portion 2 and the back sheet portion 3. The belt-shaped member 10 that is liquid permeable is disposed on the skin noncontacting side of the back sheet portion 3, and is arranged along a longitudinal direction (LD) of the absorbent article main body, on the other side of the absorbent article main body in a thickness direction (TD).

A sanitary napkin main body 5 and the belt-shaped member 10 join each other at a joining portion 8 provided at a predetermined portion in the longitudinal direction (LD). For example, the sanitary napkin main body 5 and the belt-shaped member 10 are connected to each other at the substantially central region of the sanitary napkin main body 5, where the wearer's excretory part is brought into contact with a liquid permeable region 21 of the sanitary napkin main body 5. The belt-shaped member 10 is provided along the longitudinal direction (LD) of the sanitary napkin main body 5, and extends toward the front region (F) or the rear region (R), from the joining portion 8 as a starting point. The term "front region (F)" indicates a region from the vaginal opening to the abdominal part of the wearer, when the sanitary napkin 1 is worn. The term "rear region (R)" indicates a region from the vaginal opening to the buttocks of the wearer, when the sanitary napkin 1 is worn. The term "central region" indicates a region corresponding to the wearer's excretory part, including the vaginal opening, when the sanitary napkin is worn.

In the present embodiment, a joining material is used to join the sanitary napkin main body 5 and the belt-shaped member 10; however, these may be connected to each other by applying pressure or the like.

Both end portions in the longitudinal direction (LD) of the belt-shaped member 10 extend from the outer edge portion of both end portions of the sanitary napkin main body 5 in the longitudinal direction (LD), and grip portions 6f and 6r are provided at extended portions, respectively. The skin noncontacting side of the grip portions 6f and 6r is provided with engaging portions 9f and 9r, which engage with the underwear 50 as a target object. More specifically, the engaging portions 9f and 9r are provided in both end portions of the belt-shaped member 10, in the near center in the longitudinal direction (LD).

In the present embodiment, the sanitary napkin 1 is further provided with a leak-proof groove portion 7 that can be formed by continuously compressing the top sheet portion 2 and the absorbent core portion 4. Furthermore, it should be noted that the present invention is not limited thereto, and a leak-proof wall (a so-called gather) (not shown) formed using an elastic member and a leak-proof sheet may be arranged along both sides in the width direction (WD) of the sanitary napkin.

The sanitary napkin 1 is folded in three or four with the top sheet portion inside, and individually packed in a package in the form of a packed body. An opening portion of the package is provided with a tape. The inside of the packed body of the sanitary napkin 1 can be processed with a parting agent such as silicon, in order to make tape and the like easily removable from the packed body. In addition, a release paper and the like processed with a parting agent can be provided so as to cover an adhesive portion of the sanitary napkin 1, which is used for fixing the sanitary napkin 1 to underwear or the wearer's body.

### 1-2. Sanitary Napkin Main Body

As shown in FIGS. 1 and 2, the sanitary napkin main body 5 is formed in a substantially elongated shape. The sanitary napkin main body 5 has, for example, a rectangle, an elliptical shape, and a pear-shape, as well as a shape including so-called wings W1 and W2 (described later), which prevent dislocation thereof from the underwear 50. Furthermore, the present invention may employ any shape suitable for the wearer's body and the shape of the underwear 50. The length of the sanitary napkin main body 5 in the longitudinal direction is, for example, preferably from 100 to 500 mm, and more preferably from 150 to 350 mm. The length in the width direction is, for example, preferably from 30 to 200 mm, and more preferably from 40 to 180 mm.

The sanitary napkin body 5 has a liquid permeable top sheet portion 2 that constitutes a surface layer and is disposed on the skin contacting side of a wearer, a liquid impermeable back sheet portion 3 that constitutes a back layer and is disposed on the skin noncontacting side of the wearer, and a liquid retainable absorbent core portion 4 that constitutes an absorption layer and is interposed between the top sheet portion 2 and the back sheet portion 3.

In addition, the sanitary napkin main body 5 has a liquid permeable region 21, formed in a substantially elliptical shape, in a substantially central portion in the width direction (WD) of the sanitary napkin main body 5. The liquid permeable region 21 is surrounded by the leak-proof groove portion 7, and the leak-proof groove portion 7 partitions the liquid permeable region 21. Excrement discharged from the excretory part, such as menstrual blood, passes through the top sheet portion 2 in the liquid permeable region 21, and is then absorbed by the absorbent core portion 4. Since the back sheet portion 3 disposed on the non-skin contact surface is liquid impermeable, the excrement can be absorbed and retained by the absorbent core portion 4, without reaching the skin noncontacting side. In addition, a compressed part is formed in the leak-proof groove portion 7 by disposing areas of high compression and low compression continuously or at predetermined intervals, so as to surround the liquid permeable region 21. The leak-proof groove portion 7 prevents mainly the leakage of excrement such as menstrual blood absorbed by the absorbent core portion 4, and prevents the spread of excrement in the absorbent core portion 4.

Here, the top sheet portion 2 and the absorbent core portion 4 are each adhesively joined with a hot melt adhesive. In addition, the top sheet portion 2 and the back sheet portion 3 are joined by a joining portion (not shown) formed by a hot melt adhesive and hot pressing. As a whole, the surfaces between the sheets are joined by a hot melt adhesive, and ends of the sheets are joined by the joining portion (not shown) formed by the hot pressing process.

The coating patterns for hot melt adhesion include: spiral coating, controlled seam coating, coater coating, curtain coater coating, summit-gun coating and the like, for example. The weight of the adhesive in the hot melt adhesion is preferably 1 to 30 g/m², and more preferably 3 to 10 g/m². In addition, in a pattern where the adhesive is coated linearly, the line diameter thereof is preferably from 30 to 300 µm.

### 1-3. Belt-Shaped Member

As shown in FIGS. 3 to 8, the belt-shaped member 10 is formed in a substantially elongated shape. The belt-shaped member 10 can be obtained by sandwiching a plurality of filiform elastic members 12 between a pair of belt-shaped base material sheets 11 and 13, thereby joining the base material sheets 11 and 13. More specifically, the belt-shaped member 10 can be obtained by: extending the elastic member 12 to a predetermined length by applying a predetermined tension in the longitudinal direction (LD) thereof; and sandwiching the extended elastic member 12 between the base material sheets 11 and 13. A spun bond non-woven fabric composed mainly of polypropylene (PP) can be used as the base material sheets 11 and 13, for example. In this case, the fiber weight thereof is preferably 15 to 25 g/m². The fineness thereof is preferably 1.5 to 2.5 dtex. The elastic member 12 can be, for example, an elastic yarn of polyurethane. The fineness of the elastic yarn is preferably 350 to 450 dtex. The number of the elastic yarn that can be used is from seven to nine, for example.

Thus, by sandwiching the elastic member 12 between the base material sheets 11 and 13, releasing the tension, and then releasing the extension of the elastic member 12, an extension allowance can be formed in the base material sheets 11 and 13. As a result, the belt-shaped member 10 becomes extendable. The term "extension allowance" includes sag and looseness developed in the base material sheets 11 and 13 by releasing the extension of the elastic member 12. The belt-shaped member 10 can be extended by the amount of the sag and the like. In addition, the belt-shaped member 10, in which the elastic member 12 is sandwiched between the inflexible base material sheets 11 and 13, is able to prevent so-called neck in, namely the state where the substantially central part of an elastic member and the like becomes narrow when the elastic member and the like is stretched.

Other extension processes include a partial slitting process, and preferably, a corrugated embossing finish. The corrugated embossing finish can be relatively easily obtained, by, for example, meshing the base material sheets 11 and 13 between a pair of corrugated embossing rollers. When employing the corrugated embossing finish, the belt-shaped member 10 may be formed with the elastic member 12 sandwiched between the base material sheets 11 and 13, in a state where the elastic member 12 is either stretched or not stretched.

FIGS. 6 and 7A show a specific example of the corrugated embossing finish. As shown in FIGS. 6 and 7A, the corrugated embossing finish produces a corrugated pattern where concave portions and convex portions are continuously formed in the longitudinal direction. The pitch between the convex portions is, for example, from 0.5 to 5.0 mm. The height of the convex portions is, for example, from 0.5 to 5.0 mm. The clearance between the convex side surfaces in meshing engagement is, for example, from 0 to 3.0 mm.

The belt-shaped member 10 thus formed can allow for easy setting of the extension range thereof. For example, in a case where the extension range is 130% of the non-extended state, the wearer can easily pull the belt-shaped member 10 up to 130% elongation; however, pulling more than that requires excessive force. The wearer thus can easily recognize the extension range. By thus defining the extension range, everyone can wear the absorbent article with a stable extension range, without limiting the wearer's size.

In addition, in the production method of extension allowance shown in FIG. 5, the extension allowance is randomly produced. To make the extension allowance regular, for example, an embossing pattern, partially having blanks 30 (flat portions) in the width direction (WD) in the corrugated pattern having concave and convex portions in the longitudinal direction (LD) of the base material sheets 11 and 13, as shown in FIG. 7B, can be used. More specifically, in addition to the embossing pattern shown in FIG. 5, blanks 30 having a width of 0.5 to 3.0 mm may be formed at pitches of 5 to 40 mm in the width direction (WD). Alternatively, the blanks 30 may be formed by coating a base material with a hot melt adhesive in a continuous coating pattern in the width direction (WD) and in an intermittent coating pattern in the longitudinal direction (LD). This can prevent a generation of regular creases.

Furthermore, in the present embodiment, the inextensible material is used as the base material sheets 11 and 13; however, the present invention is not limited thereto and, for example, an extensible material or a flexible material can also be used. In addition, in the present embodiment, the belt-shaped member 10 is constituted of the base material sheets 11 and 13, and the elastic member 12; however, the present invention is not limited thereto and may employ only a flexible base material sheet. For example, it is possible to employ a fibrous sheet using a thermoplastic elastomer resin. More specifically, an example of the fibrous sheet includes: a non-woven fabric obtained by blending urethane fiber and synthetic fiber; a non-woven fabric including a layer obtained by forming elastomer resin by melt blowing; and a film sheet.

The elastic member 12 is not necessarily provided in the entirety of the base material sheets 11 and 13, and may be provided in at least a part thereof. For example, a region where an engaging material is provided as the engaging portion for the underwear 50 may be constituted of the base material sheets 11 and 13, and the rest may be arranged so that the elastic member 12 is interposed between the sheets 11 and 13. In addition, the region where the grip portion 6 is provided may be constituted of the base material sheets 11 and 13, without sandwiching the elastic member 12 therebetween.

The belt-shaped member 10 is required to have a stretchable region in at least a part thereof in the longitudinal direction (LD). Preferably, the belt-shaped member 10 is flexible at a position where the sanitary napkin main body 5 is in contact with the excretory part. More preferably, the belt-shaped member 10 is flexible at a position where the sanitary napkin body 5 is adhered to the vaginal opening. In other words, the belt-shaped member 10 may partially include an unstretchable region in the longitudinal direction (LD), when being worn. For example, the region where the back sheet portion 3 and the belt-shaped member 10 are connected to each other, and the region where the sanitary napkin main body 5 or the belt-shaped member 10 is engaged with the underwear 50, may be unstretchable, and the rest may be stretchable. Furthermore, it should be noted that the joining portion 8 for joining the back sheet portion 3 with the belt-shaped member 10, and the engaging portions 9f and 9r for engaging the sanitary napkin main body 5 or the belt-shaped member 10 with the underwear 50, are preferably configured to be unstretchable. This is to prevent the belt-shaped member 10 from being disjoined from the back sheet portion 3, and to prevent the belt-shaped member 10 from disengaging from the underwear 50.

The unstretchable region may be obtained, for example, by joining the elastic member 12 in the non-extended state, with the base material sheets 11 and 13. Alternatively, the unstretchable region may be obtained by cutting the elastic member 12 in the extended state, thus eliminating a predetermined elastic force thereof. Furthermore, extensible regions generated by the embossing finish or the like may be joined with each other to form the inflexible region. The base material sheets 11 and 13 may inherently be inflexible without applying any process for forming the flexible region, such as the corrugated embossing finish of the base material sheets 11 and 13.

The length of the belt-shaped member 10 in the width direction (WD) is preferably, for example, in the range of 30 to 150% of the length of the sanitary napkin main body 5 in the width direction (WD), and more preferably in the range of 60 to 130% thereof. This is because, in a case where the length is less than 30% of the length of the sanitary napkin main body 5 in the width direction (WD), for example, the sanitary napkin main body 5 cannot sufficiently adhere to the wearer's body. Alternatively, in a case where the length is greater than 150% thereof, the area to be in contact with the thighs of the wearer is excessive, and skin irritation or the like might occur by frictional contact therewith.

In addition, the length of the belt-shaped member 10 in the longitudinal direction (LD) is preferably, for example, in the range of 30 to 300% of the length of the sanitary napkin body 5 in the longitudinal direction (LD), and can be exemplified in the range of 70 to 150% thereof. This is because, in a case where the length is less than 30% of the length of the sanitary napkin body 5 in the longitudinal direction (LD), the sanitary napkin body 5 cannot sufficiently adhere to the wearer's body, even if the belt-shaped member 10 is extended. Alternatively, in a case where the length is greater than 150% thereof, the area to be in contact with the thighs of the wearer is excessive, and skin irritation or the like might occur by frictional contact therewith. Moreover, for example, the belt-shaped member 10 in the extended state might not be attached to the underwear 50, and might not adhere to the excretory part.

The extensible range of the belt-shaped member 10 is preferably, for example, in the range of 105 to 300% of the non-extended state of the elastic member 12, and more preferably in the range of 110 to 180% thereof. This is because, in a case where the extended state is less than 105%, stress for pressing the sanitary napkin main body 5 against the wearer's body becomes weak, and it is difficult to establish the structure for lifting the sanitary napkin main body 5. Alternatively, in a case where the extended state is larger than 300%, excessive pressing force toward the wearer's body is generated, and the wearer might feel uncomfortable. Furthermore, the stress of the elastic member 12, in the extended state of 150 to 300% in extension rate, is preferably, for example, in the range of from 5 to 500 cN/25 mm, and more preferably from 20 to 100 cN/25 mm.

The belt-shaped member 10 has a grip portion 6 in each of the front region (F) and the rear region (R) in the longitudinal direction (LD). The grip portion 6 is disposed in each end of the belt-shaped member 10, so as to extend out of the outer edge of the sanitary napkin main body 5. This allows, for example, the wearer to easily recognize the grip portion 6, thereby preventing the wearer from inappropriately wearing the sanitary napkin. The grip portion 6 may be extended from the edge, to such a degree that the wearer can pinch it by fingers and the like. In addition, in order to make the grip portion 6 easier to be pinched by the wearer, a sheet member 14 having a predetermined strength may be interposed between the base material sheets 11 and 13, at a position corresponding to the grip portion 6 of the belt-shaped member 10. Furthermore, the grip part 6 being an inextensible region is preferable. 1-4. Position to Join Sanitary Napkin Main Body with Belt-Shaped Member

The sanitary napkin main body 5 and the belt-shaped member 10 are joined at the joining portion 8. The position to join the belt-shaped member 10 and the sanitary napkin main body 5 is preferably a position where the stretchability of the belt-shaped member is not required. For example, the position to join is preferably located in the vicinity of the wearer's excretory part, and more specifically the wearer's vaginal opening. The sanitary napkin main body 5 is not largely deformed and remains substantially flat in the vicinity of the excretory part in the longitudinal direction, and begins to curve along the wearer's body from the front end and the rear end of the excretory part. Thus, in the vicinity of the excretory part, the sanitary napkin main body 5 can be lifted by the belt-shaped member 10 at the front and the rear thereof extending, and a force is generated that makes the sanitary napkin main body 5 adhere to the wearer's body, even without the stretchability of the belt-shaped member 10 acting directly.

The joining range in the longitudinal direction (LD) of the sanitary napkin main body 5 and the belt-shaped member 10 is, for example, preferably a range corresponding to each of the wearer's excretory parts. More specifically, the range is, across the liquid permeable region 21 of the sanitary napkin main body 5 that is in contact with the wearer's vaginal opening, preferably no greater than 20 mm both in the front and the rear thereof in the longitudinal direction (40 mm in the longitudinal direction in total). More preferably, the range is, across the liquid permeable region 21 of the sanitary napkin main body 5 that is in contact with the wearer's vaginal opening, in the range of 1.0 mm (2.0 mm in the longitudinal direction (LD) in total) to 10 mm (20 mm in the longitudinal direction (LD) in total) both in the front and the rear thereof in the longitudinal direction. In a case where the range is greater than 20 mm in each of the front and the rear portions in the longitudinal direction (LD), the range without the stretchability of the belt-shaped member 10 is too large and the sanitary napkin main body 5 might be easily detached from the excretory part. In addition, in the width direction (WD), the range is for example in the range of 10 to 100% of the width of the sanitary napkin main body 5 in the width direction (WD). Preferably, the range is in the range of 60 to 100% thereof. In the width direction (WD), the range can be either continuously or intermittently joined.

A hot melt adhesive, embossing finish, a sonic process and the like, for example, can be exemplified as the joining material.

### 1-5. Position to Engage Belt-Shaped Member with Underwear

The belt-shaped member 10 has the engaging portions 9f and 9r to be engaged with the underwear 50 or the like, in both ends in the longitudinal direction (LD). The engaging portion 9f is engaged with the underwear 50 or the like in the front region (F), and the engaging portion 9r is engaged therewith in the rear region (R). The sanitary napkin main body 5 is not largely deformed and remains substantially flat in the vicinity of the excretory part in the longitudinal direction, and begins to curve along the wearer's body from the front end and the rear end of the excretory part. Thus, by engaging the engaging portions 9f and 9r with the underwear 50 or the like in the front (F) and the rear (R) regions, the extension range of the belt-shaped member 10 becomes greater and the sanitary napkin main body 5 is lifted along the curve of the wearer's body. Consequently, the stress from the belt-shaped member 10 is easily transferred to the liquid permeable region 21 disposed in the vicinity of the excretory part. In addition, the engaging portions 9f and 9r are preferably provided in both end portions of the belt-shaped member 10, slightly to the side of the central portion in the longitudinal direction (LD). This is to prevent the engaging material from sticking to the wearer's fingers and the like when the wearer pinches the grip portions 6f and 6r.

A hot melt adhesive, hook material, binder, and the like, for example, can be exemplified as the engaging material used for the engaging portions 9f and 9r. The engaging material used for the engaging part 9r provided in the rear region (R) may be an engaging material for engaging with the underwear 50 or an engaging material for engaging with the wearer's body. In other words, the engaging portions may be engaged either with the underwear 50 or the like, or alternatively with the wearer's body.

Furthermore, in order to prevent the belt-shaped member 10 from slipping from the underwear or the like during wearing, in a case where the belt-shaped member 10 has stretchability, the engaging force of the engaging material is required to be larger than the contractive force of the belt-shaped member 10. Accordingly, the shear stress, in other words the engaging force, is preferably set to be larger than the contractive force in the range of 5 to 500 cN/25 mm.

### 1-6. Mode of Usage

Modes of usage of the sanitary napkin 1 in the first preferred embodiment of the present invention are described with reference to FIGS. 8 and 9. The sanitary napkin 1 in the first preferred embodiment has first and second modes of usage.

The first and the second modes of usage, regarding the method of applying the sanitary napkin 1 to the underwear 50 (target object) in the wearing process of the sanitary napkin 1, are described as an example of modes of usage thereof, with reference to FIG. 8.

As the first mode of usage, the sanitary napkin 1 is first arranged at a predetermined position of the underwear 50, as shown in FIG. 8. Thereafter, a wearer holds the grip portions 6f and 6r provided in the both ends of the belt-shaped member 10, and pulls each thereof toward the front region (F) and the rear region (R), respectively. The belt-shaped member 10 is thus extended. In a case where the sanitary napkin 1 is provided with the cover member 15 (described later), the engaging portions 9f and 9r appear at this time on the skin noncontacting side of the belt-shaped member 10. After extending the belt-shaped member 10 to a predetermined degree, the wearer engages the engaging portions 9f and 9r provided in both ends of the skin noncontacting side thereof to a predetermined position on the underwear 50 being a target object. More specifically, the engaging material of the engaging portions 9f and 9r is adhered to the underwear 50. At this time, the engaging material can be exposed by removing a release paper (not shown) on the skin noncontacting side of the engaging portions 9f and 9r. Furthermore, the term "predetermined position on the underwear 50" includes, for example, an expected position where the liquid permeable region 21 of the sanitary napkin 1 can be brought into contact with the wearer's excretory part. The belt-shaped member 10 is preferably extended as much as possible to be adhered to the underwear 50.

On the completion of the attachment of the sanitary napkin 1, the underwear 50 is worn in this state so that the belt-shaped member 10 and the sanitary napkin 5 can be deformed in a gentle curve, and stress can be transferred from the belt-shaped member 10 to the sanitary napkin main body 5 toward the wearer's body. At this time, the belt-shaped member 10 lifts and pushes the sanitary napkin main body 5 against the wearer's body. As a result, the wearer's excretory part and the groove in the vicinity thereof can be in close contact with the sanitary napkin 1 (refer to FIG. 9).

In the second mode of usage, as shown in FIG. 8, the sanitary napkin 1 is arranged at the same position as in the first mode of usage. In this state, the underwear 50 is put on. Thereafter, the grip portions 6f and 6r provided on the belt-shaped member 10 are held by inserting hands between the underwear 50 and the wearer's body in each of the front region (F) and the rear region (R), and each thereof toward the front (F) and the rear region (R), respectively, are pulled along the curve of the body. Here, in a case where the sanitary napkin 1 is provided with the cover member 15 (described later), for example, the engaging portions 9f and 9r provided on the skin noncontacting side of the belt-shaped member 10 appear when the wearer pulls the grip portions 6f and 6r. However, the sanitary napkin 1 is fixed to a predetermined position on the underwear 50.

Furthermore, in a case where the engaging portion 9r is provided with a release paper, for example, the release paper may be removed in advance or, after or before pulling in the longitudinal direction.

At this time, as shown in FIG. 9A, the underwear 50 is lifted toward the wearer's body together with the sanitary napkin 1; therefore, by engaging the grip portions 6f and 6r of the belt-shaped member 10 with the underwear 50 by pulling, the position of the sanitary napkin 1 can be adjusted so that the sanitary napkin 1 better adheres to the groove in the vicinity of the wearer's excretory part.

Thus, since the wearer fixes the belt-shaped member 10 to the underwear 50 by pinching the grip portions 6f and 6r and pulling the belt-shaped member 10 toward the front (F) and the rear (R) regions, tension in the longitudinal direction can be exerted on the belt-shaped member 10. Thereafter, by bringing the belt-shaped member 10 toward the wearer's body, the belt-shaped member 10 and the sanitary napkin main body 5 can be deformed in a gentle curve, and stress can be transferred from the belt-shaped member 10 to the sanitary napkin main body 5 toward the wearer's body. At this time, the belt-shaped member 10 lifts and pushes the sanitary napkin main body 5 against the wearer's body. As a result, the wearer's excretory part and the groove in the vicinity thereof can be in close contact with the sanitary napkin 1 (refer to FIGS. 9B and 9C).

Furthermore, it should be noted that, in an alternative mode of usage in the present embodiment, the belt-shaped member 10 may not be used. In this case, the belt-shaped member 10 can be used after the underwear 50 is put on.

### 2. Other Embodiments

The second to the sixteenth embodiments of the present invention are described with reference to FIGS. 10 to 41. The second preferred embodiment shows another preferred embodiment of the belt-shaped member 10. The third embodiment shows another embodiment regarding the engaging material used for the belt-shaped member 10. The fourth to the seventh preferred embodiments show other preferred embodiments where the cover member 15 provided. The eighth embodiment shows another embodiment regarding the position where the belt-shaped member 10 is provided. The ninth preferred embodiment shows another embodiment where the belt-shaped member 10 does not have the engaging portion 9r. The tenth and eleventh preferred embodiments show other preferred embodiments regarding a guide element of the belt-shaped member 10. The twelfth to fourteenth preferred embodiments show other preferred embodiments regarding the implication of the liquid permeable region 21 of the sanitary napkin main body 5. The fifteenth and seventeenth preferred embodiments show other preferred embodiments regarding the arrangement of the belt-shaped member 10.

In the following description, the same reference numerals have been retained for similar parts that are identical to that described in the first embodiment, with the descriptions thereof omitted.

### 2-1. Second embodiment

A sanitary napkin 1P in the second preferred embodiment of the present invention is described with reference to FIGS. 10 to 14. As shown in FIGS. 10 to 14, the sanitary napkin 1P of the second embodiment is different from the first embodiment in a belt-shaped member 10P. More specifically, as shown in FIGS. 12 to 13C, the sanitary napkin 1P of the second embodiment is arranged so that the belt-shaped member 10P is constituted of the sanitary napkin body 5 and an inflexible sheet joined at the joining portion 8 on the skin noncontacting side. As the inflexible sheet, for example, a non-woven fabric or a film sheet can be used; however, any member can be used that has strength for withstanding the stress exerted when pulling up the belt-shaped member 10P. In addition, the inflexible sheet can be in the shape of a belt, a string or the like; however, a belt-shape is preferred. Thus, the sanitary napkin 1P can employ an inflexible material as the belt-shaped member 10P. Alternatively, a plurality of base material sheets of different strengths may be used.

In addition, as shown in FIG. 14, a belt-shaped member 10P of the second embodiment may have a turnback portion 17 of a predetermined size. By providing the turnback portion 17 of a predetermined size, the belt-shaped member 10P can have a predetermined length. In addition, by arranging the belt-shaped member 10P in the folded state, the belt-shaped member 10P can have a predetermined length and can be accommodated in a compact form. Furthermore, the turnback portion 17 may or may not be joined with the overlapping surface. In a case where the turnback portion 17 is joined with the overlapping surface, the joint is preferably weak for easy detachment.

The term "the predetermined length" is preferably in the range of 30 to 300% of the length in the longitudinal direction of the sanitary napkin main body 5, and more preferably in the range of 70 to 150% thereof. The predetermined size of the turnback portion 17 includes the length that permits folding back for maintaining the predetermined length of the belt-shaped member 10P.

### 2-2. Third Embodiment

A sanitary napkin 1A of the third embodiment of the present invention is described with reference to FIGS. 15 to 20B. As shown in FIGS. 15 to 20B, the sanitary napkin 1A of the third embodiment of the present invention is different from the first preferred embodiment in the arrangement of engaging material in the belt-shaped member 10.

More specifically, as shown in FIGS. 15 to 18C, the sanitary napkin 1A of the third embodiment is provided with two joining portions 8r and 8f, which are extending in the width direction (WD) and spaced apart from each other in the longitudinal direction (LD), on the skin noncontacting side of the belt-shaped member 10. In this case, in the sanitary napkin 1A of the third embodiment, the sanitary napkin main body 5A must be stretchable along with the extension of the belt-shaped member 10, in a region between the joining portions 8r and 8f. If the sanitary napkin main body 5A is not stretchable, the stretchability of the belt-shaped member 10 is eliminated in the region between the joining portions, which fix the belt-shaped member 10 to the sanitary napkin main body 5A in the portions spaced apart from each other in the longitudinal direction (LD). In addition, also in a case where the engaging material is disposed lengthwise in the longitudinal direction (LD), the stretchability of the belt-shaped member 10 is eliminated in the region.

Therefore, the sanitary napkin 1A of the third embodiment is provided with a stretchable region (S) substantially in a center of the sanitary napkin main body 5A, and joining portions 8r and 8f, which are spaced apart from each other, are arranged in both ends thereof. Thus, even in a case where the joining portions 8r and 8f, which are extending in the width direction (WD) and spaced apart from each other in the longitudinal direction (LD), are provided, the stretchable region (S) provided therebetween can make the sanitary napkin main body 5A extensible. Therefore, the sanitary napkin main body 5A can extend along with the extension of the belt-shaped member 10, and the belt-shaped member 10 is stretchable also in the region.

The stretchable sanitary napkin main body 5A' is described hereinafter. A sanitary napkin main body constituted of elastic materials, for example, can be exemplified as the stretchable sanitary napkin main body 5A'. In addition, coiled fiber such as heat-shrinkable fiber may also be used. By using elastic materials and coiled fiber such as heat-shrinkable fiber, the sanitary napkin main body 5A' can be made extensible.

In addition, even in a case where the sanitary napkin main body 5A' is constituted of non-extensible material, the sanitary napkin main body 5A' can be made extensible by an extension process. For example, as shown in FIGS. 19A and 19B, by providing a stretchable region (S) such as corrugated embossing finish in a predetermined portion of the sanitary napkin main body 5A', the sanitary napkin main body 5A' can be made extensible. FIG. 19A shows a non-extended state of the sanitary napkin main body 5A', and FIG. 19B shows an extended state thereof.

Alternatively, as shown in FIG. 20A, for example, by providing a plurality of slits 90 with predetermined cut lines in the width direction (WD) in a stretchable region (S) provided in a predetermined portion of a sanitary napkin main body 5A'', the sanitary napkin main body 5A'' can be made extensible. As shown in FIG. 20B, in a case where a predetermined stress is applied to each of the front (F) and the rear (R) regions in the longitudinal direction of the sanitary napkin main body 5A'', the slits 90 are opened by a force pulling in a longitudinal direction (LD) the cut lines provided along the width direction (WD) thereof. The sanitary napkin main body 5A'' can thus extend within the degree of opening of the slits 90 in the longitudinal direction (LD). 2-3. Fourth to Seventh Preferred Embodiments

Sanitary napkins 1B, 1C, 1D and 1E of the fourth to the seventh embodiments are described with reference to FIGS. 21 to 29B. As shown in FIGS. 21 to 29B, the sanitary napkins 1B, 1C, 1D and 1E of the fourth to the seventh embodiments are different from the first embodiment in having a cover member 15.

As shown in FIGS. 21 to 23C, the sanitary napkin 1B of the fourth embodiment has the cover member 15B covering at least a part of the belt-shaped member 10 on the skin noncontacting side of a sanitary napkin main body 5B. More specifically, the cover member 15B covers at least a part in the longitudinal direction (LD) and the entirety in the width direction (WD) of the belt-shaped member 10. The belt-shaped member 10 is disposed slidably between the sanitary napkin main body 5B and the cover member 15B, from the joining portion 8 as a starting point.

In the fourth embodiment, the cover member 15B and the sanitary napkin main body 5B are joined with each other on both side portions of the sanitary napkin 1B. More specifically, the cover member 15B is joined with the back sheet portion 3 in both side portions of the sanitary napkin 1B.

As shown in FIGS. 23A to 23C, the cover member 15B may also be joined with the back sheet portion 3 in both side portions of the sanitary napkin 1B in a projected state. In addition, as shown in FIG. 27, at least 50% of the width of the back sheet portion 3 in the width direction (WD) may be projected from both side portions, folded back, overlapped in the skin noncontacting side, and joined.

The length of the cover member 15B in the longitudinal direction (LD) is preferably, for example, in the range of 10 to 100% of the length of the sanitary napkin main body 5B in the longitudinal direction (LD), and more preferably in the range of 50 to 90% thereof. In a case where the length is less than 10% thereof, for example, the degree of freedom between the sanitary napkin main body 5B and the belt-shaped member 10B is too large, involving a risk of slipping during wearing. Alternatively, in a case where the length is greater than 100% thereof, the cover member 15 becomes an obstacle in the operation of extending and engaging the belt-shaped member 10B with the underwear 50.

The length of the cover member 15B in the width direction (WD) is preferably, for example, in the range of 30 to 150% of the length of the sanitary napkin body 5B in the width direction (WD), and more preferably in the range of 50 to 110% thereof. In a case where the length is less than 30% thereof, the sanitary napkin body 5B cannot sufficiently adhere to the wearer's body. Alternatively, in a case where the length is greater than 150% thereof, for example, the area to be contacted with the thighs is excessive, involving the risk of skin irritation due to frictional contact or the like.

The cover member 15B may be constituted of either a different material from the construction materials of the sanitary napkin main body 5B, or any one of the construction materials. In this case, a common construction material may be elongated so as to continuously form the cover member 15B. For example, an extension portion of the top sheet portion 2 or the back sheet portion 3 may be used therefor. In the case where the extension portion of the top sheet portion 2 or the back sheet portion 3 are used, the respective sheets disposed continuously may be overlapped and connected to each other on the skin noncontacting side. For example, a predetermined proportion of the length of the sanitary napkin main body 5B in the width direction (WD) may be extended, and the respective extended sheets may be folded back, overlapped, and joined, in a substantially central portion in the width direction on the skin noncontacting surface of the sanitary napkin main body 5B.

As shown in FIG. 24, the cover member 15C of the fifth embodiment may be disposed in both the front (F) and the rear (R) regions of the sanitary napkin main body 5C. Alternatively, as shown in FIG. 25A, both side portions of the cover member 15C can be folded inside the sanitary napkin main body 5C and joined with the back sheet portion 3. This arrangement allows for a large frontage between the back sheet portion 3 and the cover member 15C, for example, thereby allowing an easy expansion of the belt-shaped member 10.

In addition, as shown in FIG. 25B, an elastic member 16 may be disposed in each of the folded portions of the cover member 15C on both side portions in the width direction (WD) of the sanitary napkin main body 5C. With this configuration, for example, even if the sanitary napkin main body 5C or the belt-shaped member 10 has a tendency to twist, the elasticity of the elastic member 16 produces a force to restore the sanitary napkin main body 5C or the belt-shaped member 10 from the twist, thereby preventing the twist thereof.

As shown in FIG. 26A, the cover member 15D of the sixth embodiment can be disposed so as to cover the engaging portions 9f and 9r of the belt-shaped member 10. In this case, when the belt-shaped member 10 is not stretched, the grip portions 6f and 6r of the belt-shaped member 10 extend from the outer edge portion in the front (F) and the rear (R) region in the longitudinal direction (LD) of the sanitary napkin main body 5D, and the engaging portions 9f and 9r are covered with the cover member 15D. This prevents, for example, the engaging portions 9f and 9r from sticking to any unintentional location before the belt-shaped member 10 is stretched. In addition, the extension of the grip portions 6f and 6r allows, for example, the wearer to easily pinch the grip portions 6f and 6r, thereby improving operability.

Furthermore, a silicon resin or the like may be coated on the surface of the cover member 15D. This is preferable because the coating of the silicon resin or the like prevents the engaging portions 9f and 9r from sticking to the cover member 15D. Alternatively, the surface of the engaging portions 9f and 9r may be covered with a release paper or the like.

A plurality of cover members 15D may be provided, and are preferably arranged at least in the vicinity of the front (F) and the rear (R) region of the sanitary napkin main body 5. This can prevent slippage of the belt-shaped member 10 from the sanitary napkin main body 5 during wearing.

As shown in FIGS. 28 to 29B, in the cover member 15E of the seventh embodiment, engaging portions 91R and 91L to be engaged with the underwear 50 may be arranged in both side edges, respectively, in the substantially a central portion of a cover member 15E in the width direction (WD). With this arrangement, the underwear 50 can also be lifted to the wearer's body by the tension of the belt-shaped member 10 and, for example, prevent dislocation between the sanitary napkin 1E and the underwear 50.

As shown in FIG. 28, in the seventh embodiment, a pair of engaging portions 91R and 91L extending in the longitudinal direction (LD) is provided on both sides in the width direction (WD) of the substantially central portion of a cover member 15E; however, a plurality of engaging portions may be provided. Alternatively, the engaging portions may be provided throughout in the width direction (WD) of the cover member 15E. In addition, the engaging portions may be provided in a range from the substantially central portion to the front (F) and the rear (R) region of the cover member 15E. Furthermore, they may be arranged throughout the entirety of the cover member 15D.

### 2-4. Eighth Preferred Embodiment

A sanitary napkin 1F of the eighth embodiment of the present invention is described with reference to FIGS. 30 and 31A. As shown in FIGS. 30 and 31A, the eighth embodiment is different from the first embodiment in the location of the belt-shaped member 10.

More specifically, in the sanitary napkin 1F of the eighth embodiment, a tissue 72 is disposed between the top sheet portion 2 and the absorbent core portion 4. In addition, the belt-shaped member 10 is disposed between the tissue 73 and the absorbent core portion 4. In this case, the top sheet portion 2 and the back sheet portion 3 are joined with each other in both side portions of the sanitary napkin 1E, as shown in FIG. 24A. The belt-shaped member 10 extends from an opening, which is a portion not being joined, in each of the front (F) and the rear (R) regions of the sanitary napkin 1E. Furthermore, the belt-shaped member 10 may be disposed between the top sheet portion 2 and the tissue 72.

In the eighth preferred embodiment, the base material sheets 11 and 13 of the belt-shaped member 10 are preferably hydrophilic and liquid permeable. This can effectively facilitate mobility of excrement such as menstrual blood from the top sheet portion 2 to the absorbent core portion 4. Alternatively, the belt-shaped member 10 may be disposed between the absorbent core portion 4 and the back sheet portion 3, as shown in FIG. 24B. In addition, when an absorbent core portion (not shown) is constituted of a plurality of layers, the belt-shaped member 10 may be interposed between the layers thereof.

### 2-5. Ninth Preferred Embodiment

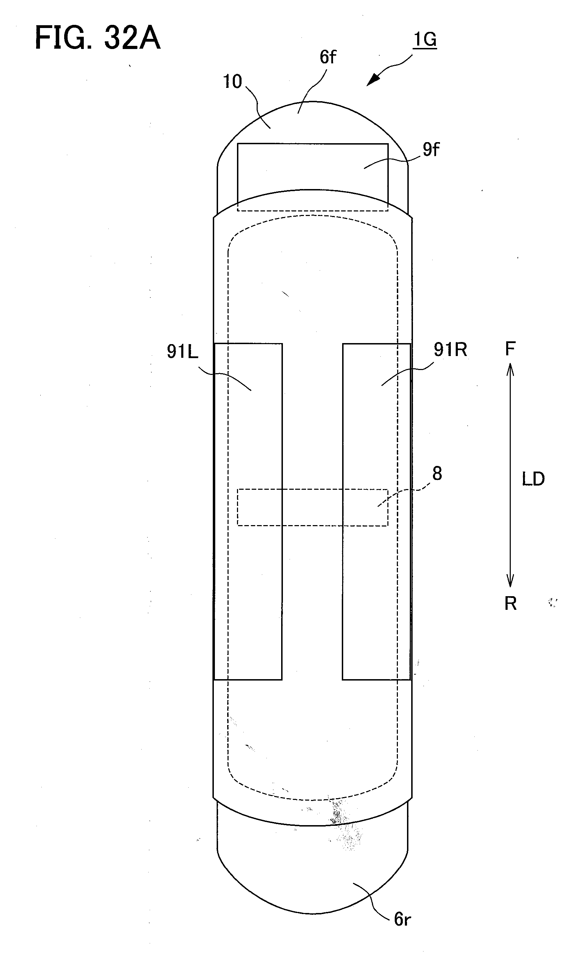

A sanitary napkin 1G of the ninth embodiment of the present invention is described with reference to FIGS. 32A and 32B. As shown in FIG. 32A, the sanitary napkin 1G of the ninth embodiment of the present invention is different from the first embodiment in that the engaging portion 9r to be engaged with the underwear 50 is not provided in a belt-shaped member 10G in the rear region (R). More specifically, in the sanitary napkin 1G of the ninth embodiment does not necessarily have the engaging portion 9r to be engaged with the underwear 50 in the rear region (R).

The following is provided as a mode of usage of this case. For example, after pulling down the underwear 50 to the wearer's knees, engaging portions 91R and 91L are engaged with the underwear 50 at both side portions in a sanitary napkin main body 5G. Thereafter, a grip portion 6f in the front region (F) is pinched and pulled toward a predetermined portion of the underwear 50, with which the grip portion is engaged. In this state, the underwear 50 is put on. Subsequently, from the back of the wearer's body, a hand is inserted between the underwear 50 and the wearer's body, the grip portion 6 is pinched, and the belt-shaped member 10G is pulled along the curvature of the wearer's body to the rear region (R) so that it is inserted into the groove in the vicinity of the excretory part, namely the groove in the vicinity of the buttocks.

Alternatively, for example, after pulling down the underwear 50 to the wearer's knees, engaging portions 91R and 91L are engaged with the underwear 50 at both side portions in a sanitary napkin main body 5G, and then the underwear 50 is put on. Subsequently, from the front face and the back of the wearer's body, hands are inserted between the underwear 50 and the wearer's body, the grip portions 6f and 6r are pinched, and the belt-shaped member 10G is pulled along the curvature of the wearer's body to the front (F) and the rear (R) region. Here, the grip portion 6f is engaged with a predetermined portion of the underwear 50, and the grip portion 6r is inserted into the groove in the vicinity of the excretory part, more specifically, the groove in the vicinity of the buttocks.

### 2-6. Tenth and Eleventh Preferred Embodiments

Sanitary napkins 1H and 1I of the tenth and eleventh embodiments of the present invention are described with reference to FIGS. 33 to 34B. As shown in FIGS. 33 to 34B, the sanitary napkins 1H and 1I related to the tenth and eleventh embodiments of the present invention are different from the first embodiment in having a guide element. More specifically, in the sanitary napkin 1H and 1I of the tenth and the eleventh embodiments, the grip portions 6Hf, 6Hr, 6If, and 6Ir may be provided with the guide element 70 that indicates the directions of extension of the belt-shaped members 10H and 10I, respectively.

An indication sign sheet with arrows, symbols, illustrations, characters, colors, color gradation or the like, and a guide element attained by the sense of touching embossing or the like, for example, can be exemplified as the guide element 70. Alternatively, the guide element 70 may be provided by a patterning process of a hot melt adhesive on the skin-contacting or the skin noncontacting surface of the grip portions 6Hf, 6Hr, 6If, and 6Ir. In a case where the guide element 70 is formed by a colored patterning process of a hot melt adhesive, the base material sheets 11 and 13 in the grip portions 6Hf, 6Hr, 6If, and 6Ir can be thereby joined with each other. Alternatively, an indication sign sheet with a guide sign or the like printed thereon may be interposed between the base material sheets 11 and 13. In addition, a guide sign or the like may be directly printed on the base material sheets 11 and 13.

In the tenth embodiment, as shown in FIG. 33, since the grip portions 6Hf and 6Hr extend from the outer edge portion in the front (F) and the rear (R) region of the sanitary napkin main body 5H in the longitudinal direction (LD), and the skin-contacting side of the grip portions 6Hf and 6Hr is exposed, for example, the wearer can easily find the grip portions 6Hf and 6Hr, thereby improving operability. Alternatively, the grip portions 6Hf and 6Hr may have a different color from the surroundings in the belt-shaped member 10H. In addition, for example, a symbol, an illustration, or a character may be used. Alternatively, the grip portions 6Hf and 6Hr may be, for example, in the shape of a triangle, a circle, an ellipse, or corrugation.

In the eleventh embodiment, as shown in FIGS. 34A and 34B, the grip portions 6If and 6Ir may have a guide element 70' for suggesting the position of the engaging portions 9f and 9r to be engaged with the underwear 50. For example, FIG. 34B illustrates the sanitary napkin 1 in which a colored sheet is arranged on the surface where the engaging material is provided at the engaging portions 9f and 9r to be engaged with the underwear 50. A colored sheet can be exemplified as the guide element 70' for suggesting the position of the engaging portions 9f and 9r. In addition, embossing, printing, colored hot melt adhesive, and the like can be exemplified as guide element 70'. Furthermore, the base material sheets 11 and 13 of the belt-shaped member 10 may be constituted of a sheet of high transparency, to make the engaging portions 9f and 9r, disposed on the back sheet portion 3, visible.

### 2-7. Twelfth to Fourteenth Preferred Embodiments

Sanitary napkins 1J, 1K, and 1L of the twelfth to the fourteenth embodiments of the present invention are described with reference to FIGS. 35A to 37. As shown in FIGS. 35A to 37, the sanitary napkins 1J, 1K, and 1L of the twelfth to the fourteenth embodiments of the present invention are different from the first embodiment in having a guide element. More specifically, the sanitary napkins 1J, 1K, and 1L may have a guide element for suggesting the liquid permeable region 21 of the sanitary napkin 1.

As shown in FIG. 35A, wings W1 and W2 may be disposed on both side portions of the sanitary napkin 1J of the twelfth embodiment, in order to suggest that the liquid permeable region 21 is located at a substantially central portion in the width direction of the wings W1 and W2.

As shown in FIG. 36, in the sanitary napkin 1K of the thirteenth embodiment, a guide element 71 having a different color may be disposed at a predetermined position on both side portions of the sanitary napkin 1K, in order to suggest that the liquid permeable region 21 is located at a substantially central portion in the width direction of the guide element 71.

As a means for suggesting the liquid permeable region 21, the position corresponding to the wearer's excretory part, as well as the positions in the front and the rear thereof may be specified.

As shown in FIG. 37, in the fourteenth preferred embodiment, a guide element 72 for suggesting the rear region (R) may be provided at a predetermined position of the grip portions 6Lf and 6Lr of a belt-shaped member 10L. The guide element 72 can be exemplified, for example, as that formed by way of embossing, colored hot melt, printing, or the like. Alternatively, a different color from the surroundings, as well as color gradation, a symbol, an illustration, a character, or a graphic symbol, may be used to imply a desired region.

### 2-8. Fifteenth to Seventeenth Preferred Embodiments

Sanitary napkins 1M, 1N, and 1P of the fifteenth to the seventeenth embodiments of the present invention are described with reference to FIGS. 38 to 41. As shown in FIGS. 38 to 41, the sanitary napkins 1M, 1N and 1P are different from the first embodiment in the arrangement of the belt-shaped member 10.

As shown in FIG. 38, the sanitary napkin 1M of the fifteenth embodiment may be provided with a pair of belt-shaped members 10Ma and 10Mb in parallel in the width direction (WD) of the sanitary napkin main body 5M, which are extended toward the front (F) and the rear (R) regions, respectively, along the longitudinal direction (LD) of the sanitary napkin main body 5M. Furthermore, although the pair of belt-shaped members 10Ma and 10Mb is provided in parallel with respect to the width direction (WD) in the sanitary napkin 1M of the fifteenth embodiment, the present invention is not limited thereto. In other words, the sanitary napkin 1M may have a plurality of bent-shaped members in parallel with respect to the width direction.

Thus, the pair of the belt-shaped members 10Ma and 10Mb may be provided on the skin noncontacting side of the sanitary napkin main body 5M so that they are joined with the back sheet portion 3 at joining portions 8Ma and 8Mb disposed at the substantially central portion of the sanitary napkin main body 5M. This allows each of the belt-shaped members 10Ma and 10Mb to move independently during walking, and to better follow the movement of the wearer's buttocks.

Alternatively, as shown in FIG. 39, the sanitary napkin 1N of the sixteenth embodiment may be provided with a pair of belt-shaped members 10Nf and 10Nr in the longitudinal direction (LD) of the sanitary napkin main body 5N, which are extended toward the front (F) and the rear (R) regions, respectively. In this case, the region between the pair of belt-shaped members 10Nf and 10Nr is preferably a stretchable region (S). In addition, the belt-shaped member 10Nr extended toward the rear region (R) may be constituted of a plurality of belt-shaped members, as in the fifteenth embodiment, for example. In other words, both or any one of the belt-shaped members 10Nf and 10Nr may be constituted of a plurality of belt-shaped members.

Furthermore, as shown in FIGS. 40 and 41, in the sanitary napkin 1Q of the seventeenth embodiment, two belt-shaped members 18 and 19 may be arranged so as to be laminated on one another in the thickness direction (TD) of the sanitary napkin main body 5Q. In this case, as shown in FIGS. 40 and 41, the sanitary napkin 1Q has a first belt-shaped member 18 and a second belt-shaped member 19. The first belt-shaped member 18 is joined with the back sheet portion 3 at a joining portion 8c in the substantially central portion. The second belt-shaped member 19 is joined with the first belt-shaped member 18 at a joining portion 8r in the rear region (R). The first belt-shaped members 18 and the second belt-shaped member 19 are covered with a cover member 15Q in the back sheet portion 3. Furthermore, the first belt-shaped member 18 has, for example, a length of about 50% of the length of the sanitary napkin body 5Q. The second belt-shaped member 19 has, for example, a length of about 95% of the length of the sanitary napkin body 5Q.

Thus, by laminating the first and the second belt-shaped members 18 and 19 onto one another, for example, the stress induced in the range from the excretory part to the anus in the rear region (R) can be further increased, thereby improving adhesion.

As an additional embodiment to the aforementioned embodiments, the belt-shaped member may be used as an auxiliary pad by applying it to the skin-contact surface of another main sanitary napkin main body. In this case, the present invention is preferably shorter in length in the width direction (WD) and in the longitudinal direction (LD) than the main sanitary napkin main body to be used therewith. Furthermore, the auxiliary pad of the present invention may be entirely constituted of a liquid permeable material, or a liquid impermeable material partially having liquid-permeable pores, in order to facilitate the transfer of menstrual blood from the auxiliary pad to another main sanitary napkin. The menstrual blood leaking out of the auxiliary pad can thus be absorbed by the main sanitary napkin. In addition, the belt-shaped member of the present invention is extended from the sanitary napkin main body and, in a case where an engaging member is provided, can be used as a belt-shaped tape for disposal of the sanitary napkin.

### 3. Components

The components of the sanitary napkin are described hereinafter.

### 3-1. Sanitary Napkin Main Body

### 3-1-1. Top Sheet Portion

The top sheet portion 2 is disposed on the wearer's body side, and is also brought into contact with the excretory part during wearing. The top sheet portion 2 may be entirely liquid permeable or partially liquid permeable. In addition, the top sheet portion 2 may be composed of either a single sheet-like member or a plurality of sheet-like members bonded together.

A woven fabric, a non-woven fabric, or a sheet material having liquid permeability such as a porous plastic sheet can be used for the top sheet portion 2. For example, as the woven fabric or the non-woven fabric, natural fibers and chemical fibers can be exemplified. More specifically, celluloses such as pulverized pulp and cotton can be exemplified as the natural fiber. In addition, regenerated celluloses such as rayon and fibril rayon; semi-synthetic celluloses such as acetate and triacetate; thermoplastic hydrophobic chemical fiber; and hydrophilized thermoplastic hydrophobic chemical fiber can be exemplified as the chemical fiber. Single fibers made of polyethylene (PE); polypropylene (PP); polyethylene terephthalate (PET); fibers obtained by graft polymerizing polyethylene (PE) with polypropylene (PP); composite fibers having core/sheath structure; and the like, for example, can be exemplified as the thermoplastic hydrophobic chemical fiber.

Furthermore, in a case where a non-woven fabric is used, dry or wet web forming such as a carding process, spun bond process, melt blown process, and air-laid process can be used. In addition, the dry and wet web forming methods may be combined. A method of bonding includes thermal bonding, needle punch, chemical bonding, and the like, without limiting thereto. Alternatively, a spun lace formed in a sheet-like shape by a spun lace process may be used.

A perforated sheet of thermoplastic resin such as polyethylene (PE), polypropylene (PP) or polyethylene terephthalate (PET); and a perforated sheet of a porous foamed material, for example, can be exemplified as the perforated plastic sheet.

Furthermore, it is preferred to use a porous plastic clouded by adding in the range of 0.5 to 10% of filler such as titanium oxide and calcium carbonate. Alternatively, a film obtained by providing pores on a thermoplastic resin film by perforation, heat embossing finish, or cutting may be used. The porous film may be combined with the non-woven fabric, and a composite sheet thus obtained may be used.

### 3-1-2. Absorbent Core Portion

The absorbent core portion 4 is required to have functions of absorbing and retaining excrement such as menstrual blood, and is preferably a bulky material that causes little chemical irritation and hardly loses its shape. For example, celluloses such as pulverized pulp and cotton, regenerated celluloses such as rayon and fibril rayon, semi-synthetic celluloses such as acetate and triacetate, particulate polymer, fibrous polymer, thermoplastic hydrophobic chemical fiber, thermoplastic hydrophobic chemical fiber subjected to hydrophilization, and air-laid pulp subjected to a chemical bond process can be used singly or in combination.

Furthermore, although no special limitation is imposed on the method of forming these materials in the absorbent core portion 4, an air-laid process, a melt blown process, a spun lace process, or a paper making process can be used, for example, and a sheet thus formed can be used.

Cellulose foam or continuous foam of synthetic resin can also be used as the absorbent core portion 4. Furthermore, a foam material or a sheet-shaped material may be pulverized and then formed in an absorbent core.

Among these, a preferred sheet-shaped absorbent core has a fiber weight of 100 to 2000 g/m², and a length in bulk of 1 to 50 mm, which can be obtained by blending 80 to 100% of pulp with no greater than 20% of particulate polymer, then coating with a tissue, followed by an embossing finish. The embossing finish is for preventing the absorbent core from losing its shape, and the embossed area is preferably in the range of 10 to 100%, and more preferably 30 to 80%.

Furthermore, an absorption sheet and a polymer sheet, for example, can be given as other examples of the material for the absorbent core portion 4. The thickness of the absorption sheet and polymer sheet are preferred to be 0.3 to 5.0 mm. An absorption sheet and a polymer sheet used for absorbent articles such as sanitary napkins may be used.

Examples of the absorption sheet include: an absorption paper; a non-woven fabric; and a pulp sheet obtained by processing fiber with a binder. Examples of the polymer sheet include: a pulverized pulp; and a sheet obtained by blending a particulate polymer in fiber and processing the mixture into a sheet. Furthermore, in the sheet thus obtained, the particulate polymer may be dispersed in the shape of a layer or in a three dimensional form.

The material used for the absorption sheet and the fiber used in the polymer sheet preferably includes: cellulose fibers such as wood pulp; regenerated celluloses such as rayon and cupra; hydrophilic synthetic fibers such as a polyvinyl alcohol fiber; a polyacrylonitrile fiber; and fibers such as polyethylene, polypropylene, polyethylene terephthalate, a polyethylene/polypropylene composite fiber, and a polyethylene/polyethylene terephthalate composite fiber, of which a surface is hydrophilized by a surface active agent. The cellulose fiber is more preferable in consideration of hydrophilic properties.

A polymer that can absorb and retain liquid at least 20 times as heavy as its own weight and can be gelatinized is preferably used for the polymer sheet. Examples of such a polymer include: starch, cross-linked carboxymethyl cellulose, polyacrylic acid and a salt thereof, polyacrylate graft copolymer and the like.

### 3-1-3. Back Sheet Portion

For the back sheet portion 3, a thermoplastic film composed mainly of polyethylene (PE) or polypropylene (PP), a permeable resin film, a sheet obtained by joining a permeable resin film with a non-woven fabric such as spun bond or spun lace, a multilayer of SMS (spun bond/melt blown/spun bond), or the like can be used. Preferable, for example, is a film composed mainly of low-density polyethylene (LDPE) resin having a fiber weight in the range of 15 to 30 g/m² for its flexibility and comfort during wearing.

In addition, in a case where a liquid impermeable sheet is used in the belt-shaped member, the same liquid permeable sheet as the top sheet portion 2 can be used as the back sheet portion 3.

### 3-1-4. Joining Portion

The top sheet portion 2 and the absorbent core portion 4 are adhesively joined with each other with a hot melt adhesive. In addition, the top sheet portion 2 and the back sheet portion 3 are adhesively joined with each other at a joining portion formed with a hot melt adhesive and by hot pressing, or the like. In the entirety, surfaces of the sheets are joined with each other with the hot melt adhesive, and edge portions of each sheet are joined by the joining portion formed by the hot pressing process. Furthermore, the joining method is not limited to the hot melt adhesion and, for example, a heat embossing finish, ultrasonic waves, etc. may also be used singly or in combination.

The coating patterns for the hot melt adhesion include, for example, spiral coating, controlled seam coating, coater coating, curtain coater coating, summit-gun coating and the like. The weight of the adhesive in the hot melt adhesion is preferably 1 to 30 g/m², and more preferably 3 to 10 g/m². In addition, in a case where adhesive is coated in a linear pattern, the diameter thereof is preferably from 30 to 300 µm. 3-2. Belt-Shaped Member

### 3-2-1. Base Material Sheet

Regarding a case in which the back sheet portion 3 is a liquid impermeable sheet, for example, the base material sheets 11 and 13 of the belt-shaped member 10 can be the liquid permeable sheet as exemplified as the top sheet portion 2. The base material sheets 11 and 13 are exemplified by a thin non-woven fabric such as a spun bond non-woven fabric, point bond non-woven fabric, and spun lace non-woven fabric, for example. This is because a thinner non-woven fabric provides enhanced smoothness of the belt-shaped member in the stretched state. In addition, in a case where a concave and convex shape is to be applied by the corrugated embossing finish described in the first embodiment, for example, a spun bond non-woven fabric composed of a continuous fiber is preferably used, in order to prevent the non-woven fabric from being broken during a corrugated embossing finish process.

In a case where the back sheet portion 3 is a liquid permeable sheet, each of the base material sheets 11 and 13 of the belt-shaped member 10 can be the liquid impermeable sheet as exemplified above as the back sheet portion 3. The liquid impermeable sheet may be disposed only on the skin-contacting side with respect to the elastic member, or disposed on both the skin-contacting and the skin noncontacting sides.

### 3-2-2. Elastic Member

Examples of the elastic member 12 include, for example, an elastic yarn of natural rubber or polyurethane. More specifically, foams of an elastomer component or polyethylene foam can be used singly; alternatively, a string, a belt or a sheet obtained by processing a mixture including thereof can be used.

Examples of the elastomer component include: a thermoplastic elastomer of polyester, urethane, olefin, styrene, or polyamide; low density polyethylene using metallocene catalyst; and ethylene-α-olefin copolymer. These can be used singly or in combination.

As the polyester elastomer, for example, one having a hard segment of aromatic polyester and a soft segment of non-crystal polyether or aliphatic polyester can be exemplified.

As the urethane elastomer, for example, polyurethane can be exemplified, which is composed of polyester, low molecular weight glycol, and methylene bisphenyl isocyanate, in which polylactone ester polyol is addition polymerized with polyisocyanate in the presence of short chain polyol.

As the olefin elastomer, for example, ethylene-α-olefin random copolymer and one copolymerized with diene as a third composition can be exemplified.

As the styrene elastomer, block copolymers such as SEBS, SIS, SEPS, and SBS can be exemplified.

As the polyamide elastomer, for example, one of which a hard segment is nylon and a soft segment is polyester or polyol can be exemplified.

In addition, in order to stabilize the formation of the elastic member, the constitutive polymer of the elastomer component may contain, for example, high density polyethylene, low density polyethylene, linear low density polyethylene or the like. Furthermore, it may combine a blocking inhibitor, ultraviolet absorbing agent, thickening and branching agent, flatting agent, coloring agent, and other conditioning agents. Among these, the polyurethane elastic yarn is preferred for its effects related to resistibility against heat and strain.

### 3-2-3. Joining Material

As the joining material in joining the base material sheets 11 and 13 with the elastic member 12, or joining the base material sheet 11 with the base material sheet 13, for example, a heat embossing finish, ultrasonic waves, a hot melt adhesive, etc. can be used singly or in a combination thereof. For example, a hot melt adhesive is applied to the base material sheet 11 by a coating method such as spiral coating, coater coating, curtain coater coating, or summit gun coating, the elastic member 12 is laid thereon, and then the base material sheet 13 is laid thereon and joined together. In order to prevent the elastic member 12 from separating from the base material sheets 11 and 13, the elastic member 12 may be coated in advance by a coating method such as slit coating or control seam coating.

Examples of the hot melt adhesive include, for example: pressure sensitive adhesives and thermo-sensitive adhesives, each composed mainly of rubbers such as SEBS, SBS, and SIS or olefins such as linear low-density polyethylene; and water-sensitive adhesives of polyvinyl alcohol, carboxyl methyl cellulose, or gelatin, each being composed of water soluble high polymer, or of polyvinyl acetate or polyacrylic acid sodium, each being composed of a water swelling high polymer. It is preferable to use, for example, a heat sensitive adhesive that, even if the aforementioned adhesive effuses, has no tackiness at that point in time. As a more specific example, such an adhesive is exemplified by a melt-mixture in the range of 5 to 25% of SEBS, 40 to 60% of alicyclic saturated hydrocarbon, 1 to 10% of aromatic denaturated terpene, and 15 to 35% of additive.

### 3-3. Engaging Material

Examples of the engaging material for the engaging portion for engaging the sanitary napkin body to the underwear include, for example, a hot melt adhesive. As a hot melt adhesive, for example, having inherent tackiness is preferred, and a pressure-sensitive adhesive can be exemplified. The main component of the adhesive is the same as in the abovementioned joining material; more specifically, examples thereof include a melt-mixture of 15 to 25% of SEBS, 15 to 35% of plasticizer, and 40 to 70% of an adhesive-imparting agent. In addition, an oxidation inhibitor and a fluorescence inhibitor may be added in the range of 0.1 to 1.0%. The basis weight is from 10 gsm to 200 gsm, and coated uniformly or in a streak pattern or in dots, by coater coating or bead coating. Alternatively, an acrylic adhesive may be used. Furthermore, the engagement may be attained by using a plurality of hook parts that stand on the surface of each tape-shaped portion.

More specifically, the tape-shaped portion can be formed by extrusion molding of a thermoplastic synthetic resin such as polypropylene, followed by cutting and removal of a rib structure part integrally formed with the tape-shaped portion. In addition, a hook part may be formed by cutting from a side a monofilament loop composed of thermoplastic synthetic resin which is provided on the surface of the tape-shaped portion. Furthermore, the end face of the hook part may be rounded in order to eliminate the risk of damaging the skin. In order to eliminate the risk of damaging the skin, the hook part may have a rounded shape. More specifically, the top of the hook can be rounded with the shape of a die. Furthermore, no special limitation is imposed on the cross-sectional shape of the hook part, and it may be tapered or of T-shape.

On the other hand, examples of engaging material to be fixed to the wearer's body and not to the underwear 50, in a case where the engaging material is provided on the skin-contacting side of the belt-shaped member 10, include a water-soluble polymer, cross-linking agent, plasticizer, gel adhesive composed of water, and the like. More specifically, gelatin, polyacrylic acid sodium, polyvinyl alcohol, and carboxyl methyl cellulose can be exemplified as the water-soluble polymer. Water-soluble metallic salts such as calcium chloride and magnesium sulfate. Example of the plasticizer includes glycerine, wax, and paraffin can be exemplified as the cross-linking agent.

Furthermore, with respect to the pressure-sensitive adhesive and the engaging portion 9, the part having tackiness is preferably covered with a sheet obtained by coating a silicon resin on thin paper, in other words generally available release paper, or a sheet obtained by coating silicon resin on a film. This can protect the adhesive portion against dirt or release during storage.

## Claims

1. An absorbent article having a substantially elongated shape, comprising:
an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and
a belt-shaped member disposed on the second side of the absorbent article main body, along a longitudinal direction of the absorbent article main body,
wherein an engaging portion is provided in at least one end side of both ends in the longitudinal direction of the belt-shaped member.

2. The absorbent article according to claim 1, wherein the belt-shaped member is formed to be at least partially extensible.

3. The absorbent article according to claim 1, wherein the belt-shaped member is formed to be at least partially stretchable.

4. The absorbent article according to claim 2 or 3, wherein a length of the belt-shaped member in an extended state thereof is greater than a length in the longitudinal direction of the absorbent article main body.

5. The absorbent article according to claim 2 or 3, wherein a length of the belt-shaped member in a non-extended state thereof is greater than a length in the longitudinal direction of the absorbent article main body.

6. The absorbent article according to any one of claims 1 to 5, wherein a grip portion, of which at least a part extends from an outer peripheral portion in both end sides of the absorbent article main body in the longitudinal direction, is formed in each of the both end sides of the belt-shaped member in the longitudinal direction.

7. The absorbent article according to any one of claims 1 to 6, wherein a cover member is further provided on the second side of the absorbent article main body, which encapsulates at least a part in the longitudinal direction of the belt-shaped member and an entirety in the width direction of the belt-shaped member.

8. The absorbent article according to any one of claims 1 to 7, wherein the belt-shaped member joins the absorbent article main body in a predetermined position on the second side of the absorbent article.

9. The absorbent article according to any one of claims 1 to 8, wherein the grip portion has a guiding element for implying an extension direction of the belt-shaped member.

10. The absorbent article according to any one of claims 1 to 9, wherein a predetermined engaging portion is disposed in each of both ends of the belt-shaped member in the longitudinal direction.

11. The absorbent article according to any one of claims 1 to 10, wherein the belt-shaped member comprises at least one portion that is a liquid impermeable material.

12. The absorbent article according to any one of claims 1 to 11, wherein a length of the belt-shaped member in a width direction is at least 30% of a length of the absorbent article main body in a width direction.

13. The absorbent article according to any one of claims 1 to 12, wherein the belt-shaped member comprises a belt-shaped base material and an elastic member.

14. An absorbent article having a substantially elongated shape, which is used in contact with a wearer's body by being placed on an inner surface of a predetermined clothing, comprising:
an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and
a belt-shaped member disposed on the second side of the absorbent article main body, along a longitudinal direction of the absorbent article main body; and
an engaging portion provided in at least one end side of both ends in the longitudinal direction of the belt-shaped member,
wherein the belt-shaped member pushes up the absorbent article main body to the first side during wearing, by being extended and engaging the engaging portion to a target object.

15. An absorbent article having a first mode of usage and a second mode of usage, comprising:
an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion;
a belt-shaped member disposed on the second side of the absorbent article main body, along a longitudinal direction of the absorbent article main body; and
an engaging portion provided in at least one end side of both ends in the longitudinal direction of the belt-shaped member,
wherein: in the first mode of usage, the absorbent article is used with the belt-shaped member being engaged to a target object; and
in the second mode of usage, the absorbent article is used with the belt-shaped member not being engaged to the target object.

16. The absorbent article according to claim 15, wherein, in the first mode of usage, the belt-shaped member pushes up the absorbent article main body to one side during wearing, by being extended and engaged to the target object.

17. An absorbent article having a substantially elongated shape, comprising:
an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and
a belt-shaped member that is liquid permeable disposed between the top sheet portion and the absorbent core portion so as to be slidable in a longitudinal direction of the absorbent article main body,
wherein an engaging portion is provided in at least one end side of both ends in the longitudinal direction of the belt-shaped member.

18. The absorbent article according to claim 17, wherein the belt-shaped member is formed to be at least partially extensible.

19. The absorbent article according to claim 17, wherein the belt-shaped member is formed to be at least partially stretchable.

20. An absorbent article having a substantially elongated shape, comprising:
an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and
a pair of belt-shaped members, each of which is disposed on the second side of the absorbent article main body, along a longitudinal direction of the absorbent article main body,
wherein: each of the pair of belt-shaped members joins the absorbent article main body in a predetermined position on the second side of the absorbent article; and
an engaging portion is provided in at least one end side of both ends in the longitudinal direction of the each of the pair of belt-shaped members.

21. An absorbent article having a substantially elongated shape, comprising:
an absorbent article main body at least including a top sheet portion that is at least partially liquid permeable and disposed on a first side in a thickness direction of the absorbent article, and a liquid retentive absorbent core portion being disposed on a second side in the thickness direction of the absorbent article, which is the second side of the top sheet portion; and
a pair of belt-shaped members, each of which is disposed on the second side of the absorbent article main body, extending in an opposite direction to each other in the longitudinal direction of the absorbent article main body,
wherein: a first end in a longitudinal direction of each of the pair of belt-shaped members joins the absorbent article main body in a predetermined position on the second side of the absorbent article; and
an engaging portion is provided in a second end in the longitudinal direction of the each of the pair of belt-shaped members.
